# EUROPEAN PATENT SPECIFICATION

(11) **EP 1 255 541 B1**
(45) Date of publication and mention of the grant of the patent: **09.11.2005**
(21) Application number: 01911580.7
(22) Date of filing: 02.02.2001
(51) Int. Cl.: A61K 31/155, A61P 31/18, A61K 31/22, A61P 31/04

(54) **AROMATIC GUANYLHYDRAZONE FOR THE TREATMENT OF RESISTANT VIRAL INFECTIONS**
AROMATISCHES GUANYLHYDRAZON ZUR BEHANDLUNG RESISTENTER VIRALER INFEKTIONEN
GUANYLHYDRAZONE AROMATIQUE POUR LE TRAITEMENT DES INFECTIONS VIRALES RESISTANTES

(30) Priority: 02.02.2000 US 179795 P; 02.02.2000 EP 00102050
(43) Date of publication of application: 13.11.2002
(73) Proprietor: Bevec, Dorian, 82110 Germering (DE)
(72) Inventor: Bevec, Dorian, 81369 München (DE); HAUBER, Joachim, 20148 Hamburg (DE); OBERT, Sabine, 81475 Munich (DE); KERI, Gyorgy, H-1021 Budapest (HU); ORFI, Laszlo, H-1161 Budapest (HU); SZEKELY, Istvan, H-Budapest (HU); CHOIDAS, Axel, 81249 Munich (DE); BACHER, Gerald, 82110 Germering (DE)
(74) Representative: Salgo, Reinhold Caspar
(86) International application number: PCT/EP2001/001126
(87) International publication number: WO 2001/056553

(56) References cited:
- WO-A-98/20868
- US-A- 5 854 289
- US-A- 5 859 062
- US-A- 6 008 255
- COHEN P S ET AL: "The critical role of p38 MAP kinase in T cell HIV -1 replication." MOLECULAR MEDICINE, (1997 MAY) 3 (5) 339-46. , XP001018447
- KERLUND K ET AL: "Anti-inflammatory effects of a new tumour necrosis factor-alpha (TNF-alpha) inhibitor ( CNI - 1493 ) in collagen-induced arthritis (CIA) in rats." CLINICAL AND EXPERIMENTAL IMMUNOLOGY, (1999 JAN) 115 (1) 32-41. , XP001021637
- D'SOUZA M J ET AL: "Prevention of lethality and suppression of proinflammatory cytokines in experimental septic shock by microencapsulated CNI-1493." JOURNAL OF INTERFERON AND CYTOKINE RESEARCH, (1999 OCT) 19 (10) 1125-33. , XP001018446
- CAVALLINI G ET AL: "Antibacterial agents. Some new guanylhydrazone derivatives." J MED PHARM CHEM, (1961 JUL 1) 4 177-82., XP001021572
- MIDORIKAWA Y ET AL: "Evaluation of the antimicrobial activity of methylglyoxal bis( guanylhydrazone ) analogues, the inhibitors for polyamine biosynthetic pathway." JOURNAL OF APPLIED BACTERIOLOGY, (1991 APR) 70 (4) 291-3. , XP001018448

## Description

The present invention provides aromatic guanylhydrazone compounds and their use as pharmaceutically active agents, especially for prophylaxis and treatment of virally caused diseases and infections, including opportunistic infections. The guanylhydrazone compounds are also useful as inhibitors of deoxyhypusine synthase and as inhibitors for nuclear export in infectious diseases. In addition, methods for treating virally induced infections and diseases are disclosed together with pharmaceutical compositions useful within said methods.

### HIV and AIDS: History

Deaths from what has since been recognized as HIV infection with immune failure have been seen clinically, without being understood, for at least 35 years, and probably much longer. An HIV-infected British sailor, who had traveled widely, is thought to have died with severe immune deficiency and HIV infection in 1959, the earliest proven case of modern AIDS.

The genetic material of the most common HIV-1 strain is most similar to that of a virus known to naturally infect chimpanzees, and it may be that HIV's ancestors have been present in Africa, perhaps even in humans, for a very long time, perhaps thousands of years. In West Africa, a close cousin of the HIV-1, called HIV-2, is almost identical to several indigenous African monkey viruses, and almost certainly has been derived from them quite recently in virus evolutionary time (less than several centuries).

First, the term "AIDS" stands for Acquired Immune Deficiency Syndrome, but it has always been understood that the amount of immune deficiency we are interested in, is not a trivial one. AIDS is the name historically chosen for a new medical syndrome which is essentially 100% fatal, and thus in defining "AIDS" we are looking for people with an immune deficiency in the range which is life-threatening, and which will continue to grow relentlessly worse until life is impossible. One way to define immune deficiency is to define it by so-called "opportunistic infections", diseases rarely seen in people whose immune system is fully functional. In the early days of AIDS, before HIV was discovered, the syndrome was indeed defined using such opportunistic diseases.

Early in the history of AIDS, it was found that the immune defect in this disease is peculiar, and that it most visibly involves a particular kind of cells in blood and lymphatic tissues (lymph nodes), called "T-lymphocytes". In the syndrome of AIDS, certain T-lymphocytes gradually disappear from both blood and lymph tissues, and a simple T-lymphocyte count in the blood can tell how serious the reduction has been in both places (since blood lymphocytes come from the lymphatic organs). The arm of the immune system which is controlled most directly by T-lymphocytes (the body's defense against viruses and fungi) is most defective in AIDS, and viral and fungal infections are the main opportunistic infections which appear and cause death in AIDS.

After HIV was identified, statistical studies quickly picked it as the most likely answer yet proposed for the cause of AIDS. HIV has the typical lentivirus structure, identical to that of FIV and SIV. People infected with HIV were found to develop antibodies to it in the blood usually a month or two after infection, and keep them for life. The presence of antibodies in the blood for years did not seem to keep people with HIV from eventually becoming very ill, however. Today, it is known that lentiviruses are able to mutate to escape host antibodies, so that antibodies found in the same blood with these viruses often do not neutralize them, especially late in the course of disease [J. Acquir. Immune. Defic. Syndr. 7:211-219, 1994]. The HIV preferably infects CD4+ cells which get lost in AIDS patients.

Most people, however, apparently recovered completely after initial HIV infection, and felt well. As with other lentiviruses, the long latency period of HIV before secondary disease was striking. In a person with AIDS, up to 13% of lymphocytes and monocytes in the blood were found infected [N Engl J Med 326:1385-1391, 1992]. From 93% to 100% of the time, infectious virus and viral DNA could be recovered from the blood of asymptomatic people who were HIV-positive, and 100% of the time from people with AIDS [N. Engl. J. Med. 321:1621-5, 1989; AIDS 6:373-377, 1992; AIDS 8:895-900, 1994]. Higher levels of virus could be cultured from the lymphatic tissues of such people, where it was multiplying actively, even in people who appeared healthy [Clin Res 40:333, 1992; Proc Natl Acad Sci USA 88:9838-9842, 1991; J Infect Dis. 164:1051-1057, 1991; J Acquir Immune Defic Syndr 6:655-662, 1993]. Like SIV-infected monkeys and FIV-infected cats doomed to future immune failure, HIV-positive people were still infected, and most were still slowly losing immune cells in lymphatic tissues.

Opportunistic infections often also appeared, such as *candida* yeast infection in the throat (often the first infection), or the unusual fungal pneumonia caused by *Pneumocystis carinii* (this organism is particularly suppressed by CD4+ lymphocytes, and so is one of the first infections to arrive when their numbers fall). With the appearance of these markers of secondary disease, now the infected person was said to have "full-blown AIDS," or simply, AIDS.

### Lentiviruses

The spread of lentiviruses in nature involves a complex bio-ecologic system in which host, parasite, and a range of social and environmental problems interact. The viruses are disseminated exclusively by exchange of body fluids. All factors that facilitate such exchange on a large scale may potentiate epidemics and epizootics.

Similar criteria of viral genomic organization, morphology, and biological properies let to the inclusion of human immunodeficiency virus (HIV) into the lentivirus family. Originally called lymphadenopathy-associated virus (LAV), human T-cell lymphotropic virus III (HTLV-3), or AIDS-associated retrovirus (ARV) by the respective pioneers in the field, the name HIV was substituted by the Virus Nomenclature Committee. Subsequent new inductees into the lentivirus family, based on similar criteria, were the simian, feline, and bovine viruses. All these agents were called immunodeficency viruses (following the precedent of HIV) irrespective of whether they caused immunodeficency or, for that matter, any disease at all. The following table 1 gives an overview of the members of the lentivirus family known by now.

**Table 1:**

| Lentiviruses | | |
|---|---|---|
| **Virus** | **Host** | **Diseases** |
| EIAV | Horse | anemia, wasting |
| VMV | Sheep | pneumonia, wasting, arthritis, mastitis, encephalitis |
| CAEV | Goat | arthritis, mastitis, encephalitis |
| BIV | Cattle | none |
| FIV | Cat | immunodeficency, encephalitis, wasting |
| SIVs | various African monkey species | none |
| HIV-1, HIV-2 | Humans | immunodeficency, pneumonia, encephalopathy, wasting, gastroenteropathy, nephropathy |

SIV and FIV were discovered several years after HIV and the lesson that remains from the SIV and FIV studies is that lentiviruses alone in some circumstances are quite capable of causing slow immunosuppressive death.

FIV is called "Cat AIDS". The feline immunodeficiency virus (FIV) is a lentivirus discovered in Petaluma, California in 1986, where it was first obtained from the blood of two domestic cats living in a household in which there had been a number of deaths of cats from a strange immune deficiency disorder.

With respect to the numbers of HIV-infected individuals, each day about 16,000 people worldwide are infected with the virus, with 95% of new cases ocurring in developing countries (mostly Africa). More than 2.6 milion people died of AIDS in 1999 alone, the highest reported number since data gathering about the disease began in the early 1980s. According to the American Medical Association, about 45 million people worldwide are infected with HIV. Of the more than 2.6 million people died of AIDS-associated diseases in 1999, an estimated 550,000 were children under 15 years of age. Although life expectancy increased very significantly by introduction of new treatments, at the present time no cure exists for AIDS.

### Hepatitis B Virus (HBV)

Hepatitis is an inflammation of the liver that is most often caused by infection with one of five viruses, hepatitis A, B, C, D or E. In cases, particularly in those related to hepatitis B and C, "chronic hepatitis" may result. Chronic hepatitis occurs when the body is unable to completely clear the virus even though the symptoms may not persist. Continued presence of the virus over a number of years can lead to cirrhosis (scarring of the liver) or hepatocellular carcinoma (liver cancer). It is estimated that approximately 350 million people worldwide are infected with chronic hepatitis B. HBV is transmitted through sexual contact, vertical transmission (mother to child at birth) or by coming into contact with contaminated blood. It is estimated that over 2 billion people worldwide have been infected with hepatitis B virus. Of these 2 billion, approximately 350 million people have developed chronic HBV infection, putting them at high risk of developing cirrhosis and liver cancer (World Health Organization Fact Sheet on Hepatitis B, Nov. 1998).

| | |
|---|---|
| **INCIDENCE** | **140,000-320,000 infections/year in United States** |
| **PREVALENCE** | **Estimated 1-1.25 million chronically infected Americans** |
| **COSTS** | **Estimated $700 million (1991 dollars)/year (medical and work loss)** |

Chronic carriers of the HBV have been defined as those who are HBV surface antigen positive for greater than 6 months. Approximately 5-10% of those people who are infected with the virus will become carriers, an estimated 5-10% of those people infected each year will progress to chronic liver disease, cirrhosis and possibly liver cancer. About 5,000 people die in the United States each year related to HBV, 1,000 die of HBV-related liver cancer. The incubation period of HBV usually lasts from 2 to 4 months, although it may be very short (10 days) or extremely long (9 months). Before the outbreak of the acute disease HBs- and HBe-antigen are detectable in the patient's serum. The onset of acute hepatitis is characterized by the occurrence of anti-HBc antibodies which at first exclusively belong to the IgM class. From this time on anti-HBc antibodies will be detectable in the patient's serum for the rest of his life, no matter whether there is an acute hepatitis B, a form of persisting virus infection or some naturally acquired immunity to HBV.

The most significant event indicating a chronic course of hepatitis B is the absence of the HBsAg/anti-HBs seroconversion. If this phenomenon has not occurred within 6 months after the onset of the disease, persistence of the HBV infection and the related clinical pictures (asymptomatic HBsAg carrier, chronic hepatitis, cirrhosis, or hepatoma) have to be reckoned with.

While monotherapy treatment with either Epivir-HBV (lamivudine, 3TC) or Intron A (interferon-alfa) shows some benefits for chronic hepatitis B, there is a need for additional therapies. Currently, drugs that are in development include Coviracil/FTC, DAPD, L-FMAU (Triangle Pharmaceuticals), adefovir dipivoxil, tenofovir (Gilead Sciences), epavudine, epcitabine (Novirio Pharmaceuticals), as well as lobucavir, Famvir/penciclovir, entecavir/BMS-200475, racivir, DXG, L-ddA prodrug, HDP-P-acyclovir, LM-019c, CS-1091, PS-019, PS-018, ara-AMP prodrugs, thymosin, (-)-Carbovir, hammerhead ribozymes, and glycosidase inhibitors. Therefore, new antiviral drugs are highly needed.

### References HBV:

Block T et al.: Glucosidase/protein folding inhibitors as possible mutation-proof anti-hepatitis B and C virus agents. Abstract and oral presentation 16 at the 3rd International Conference on Therapies for Viral Hepatitis. December 12-16, 1999; Maui, USA and *Antiviral Therapy* 1999; 4 (Supplement 4), 8.

Cretton-Scott E et al.: Pharmacokinetics of beta-L-thymidine and beta-L-2'-deoxycytidine in woodchucks and monkeys. Abstract and poster presentation 124 at the 3rd International Conference on Therapies for Viral Hepatitis. December 12-16, 1999; Maui, USA and *Antiviral Therapy* 1999; 4 (Supplement 4), 50.

Hernandez B et al.: Cellular kinases involved in the phosphorylation of beta-L-thymidine and beta-L-2'-deoxycytidine, two specific anti-hepatitis B virus agents. Abstract and poster presentation 123 at the 3rd International Conference on Therapies for Viral Hepatitis. December 12-16, 1999; Maui, USA and *Antiviral Therapy* 1999; 4 (Supplement 4), 49.

Hostetler KY et al.: Oral activity of 1-0-hexadecyl-propanediol-3-P-acyclovir in woodchucks with chronic woodchuck hepatitis infection and synergy with lamivudine in vitro. Abstract and poster presentation 80 at the 3rd International Conference on Therapies for Viral Hepatitis. December 12-16, 1999; Maui, USA and *Antiviral Therapy* 1999; 4 (Supplement 4), 29.

Juodawlkis A et al.: Antiviral activity of beta-L-thymidine and beta-L-2'-deoxycytidine in the woodchuck model of chronic hepatitis B infection. Abstract and poster presentation 97 at the 3rd International Conference on Therapies for Viral Hepatitis. December 12-16, 1999; Maui, USA and *Antiviral Therapy* 1999; 4 (Supplement 4), 37.

Ono-Nita SK et al.: Screening of new antivirals for wild-type hepatitis B virus and three lamivudine-resistant mutants. Abstract and poster presentation 89 at the 3rd International Conference on Therapies for Viral Hepatitis. December 12-16, 1999; Maui, USA and *Antiviral Therapy* 1999; 4 (Supplement 4), 33.

Placidi L et al.: Cellular pharmacology of beta-L-thymidine and beta-L-2'-deoxycytidine in HepG2 cells and primary rat, monkey and human hepatocytes. Abstract and poster presentation 122 at the 3rd International Conference on Therapies for Viral Hepatitis. December 12-16, 1999; Maui, USA and *Antiviral Therapy* 1999; 4 (Supplement 4), 48.

Sommadossi J-P et al.: Anti-hepatitis B virus specific L-nucleosides. Abstract and oral presentation 19 at the 3rd International Conference on Therapies for Viral Hepatitis. December 12-16, 1999; Maui, USA and *Antiviral Therapy* 1999; 4 (Supplement 4), 8.

Ying C et al.: Inhibition of the replication of the DNA polymerase M550V variant of hepatitis B virus by adefovir, tenofovir, L-FMAU, DAPD, penciclovir and lobucavir. Abstract and poster presentation 76 at the 3rd International Conference on Therapies for Viral Hepatitis. December 12-16, 1999; Maui, USA and *Antiviral Therapy* 1999; 4 (Supplement 4), 27.

### Herpes simplex virus type 1 (HSV-1)

Herpes simplex virus is a highly adapted human pathogen with an extremely rapid lytic replication cycle, and yet with the ability to invade sensory neurons where highly restricted gene expression occurs with the absence of cytopathology.

Such latent infections are subject to reactivation whereby infectious virus can be recovered in peripheral tissue enervated by the latently infected neurons following a specific physiological stress. A major factor in these "switches" from lytic to latent infection and back involves the interaction between viral promoters, the viral genome, and cellular transcriptional machinery. Herpes simplex virus types 1 and 2 are generally associated with mucocutaneous facial infection (herpes labialis), genital infection (herpes genitalis), ophthalmic infection (herpes keratitis) and neonatal infection. The lesions are generally self-healing, but the infection remains, with the virus establishing latent infection in the neurons. In immunocompromised hosts, recurrence of HSV-1 may cause progressive fulminant lesions that are refractory to current antiviral therapy. Neonatal infection is usually systematically spread and may lead to encephalitis. Current antiviral therapy like Acyclovir does not eliminate viral infection. New therapies are needed to either eliminate latent virus or to suppress lesion development over the long term.

### Epstein-Barr virus (EBV)

Epstein-Barr virus, frequently referred to as EBV, is a member of the herpesvirus family and one of the most common human viruses. The virus occurs worldwide, and most people become infected with EBV sometime during their lives. In the United States, as many as 95% of adults between 35 and 40 years of age have been infected. Infants become susceptible to EBV as soon as maternal antibody protection (present at birth) disappears. Many children become infected with EBV, and these infections usually cause no symptoms or are indistinguishable from the other mild, brief illnesses of childhood. In the United States and in other developed countries, many persons are not infected with EBV in their childhood years. When infection with EBV occurs during adolescence or young adulthood, it causes infectious mononucleosis 35% to 50% of the time.

Symptoms of infectious mononucleosis are fever, sore throat, and swollen lymph glands. Sometimes, a swollen spleen or liver involvement may develop. Heart problems or involvement of the central nervous system occurs only rarely, and infectious mononucleosis is almost never fatal. There are no known associations between active EBV infection and problems during pregnancy, such as miscarriages or birth defects. Although the symptoms of infectious mononucleosis usually resolve in 1 or 2 months, EBV remains dormant or latent in a few cells in the throat and blood for the rest of the person's life. Periodically, the virus can reactivate and is commonly found in the saliva of infected persons. This reactivation usually occurs without symptoms of illness.

EBV also establishes a lifelong dormant infection in some cells of the body's immune system. A late event in a very few carriers of this virus is the emergence of Burkitt's lymphoma and nasopharyngeal carcinoma, two rare cancers that are not normally found in the United States. EBV appears to play an important role in these malignancies, but is probably not the sole cause of disease.

Most individuals exposed to people with infectious mononudeosis have previously been infected with EBV and are not at risk for infectious mononucteosis. In addition, transmission of EBV requires intimate contact with the saliva (found in the mouth) of an infected person. Transmission of this virus through the air or blood does not normally occur. The incubation period, or the time from infection to appearance of symptoms, ranges from 4 to 6 weeks. Persons with infectious mononucleosis may be able to spread the infection to others for a period of weeks. However, no special precautions or isolation procedures are recommended, since the virus is also found frequently in the saliva of healthy people. In fact, many healthy people can carry and spread the virus intermittently for life. These people are usually the primary reservoir for person-to-person transmission. For this reason, transmission of the virus is almost impossible to prevent.

The clinical diagnosis of infectious mononucleosis is suggested on the basis of the symptoms of fever, sore throat, swollen lymph glands, and the age of the patient. Usually, laboratory tests are needed for confirmation. Serologic results for persons with infectious mononucleosis include an elevated white blood cell count, an increased percentage of certain atypical white blood cells, and a positive reaction to a "mono spot" test. There is no specific treatment for infectious mononucleosis, other than treating the symptoms. No antiviral drugs or vaccines except Ganciclovir are available.

### Human herpesvirus 8 (HHV8), or Kaposi's sarcoma-associated herpesvirus (KSHV)

KSHV is a virus belonging to the family of herpesviruses. KSHV was discovered in 1994. The virus has two names that are commonly used: KSHV which is its descriptive name and HHV8 which is its formal name. Both names are essentially interchangeable. KSHV causes a blood vessel cancer called Kaposi's sarcoma (KS), a lymphoma (a cancer of the lymphocyte) called body cavity-based lymphoma and some forms of severe lymph node enlargement, called Castleman's disease. The rates of KSHV infection in the general population of Mediterranean countries (Italy, Greece, Israel, Saudi Arabia) are higher than in North America and Northern Europe. Adult populations in some portions of Africa have very infection rates (>50%). In several respects the global patterns of KSHV infection appear to be similar to those of the hepatitis B virus although we are still at an early stage of understanding the epidemiology of KSHV. This virus can be transmitted through sexual contact. KSHV is also probably transmitted to some extent through nonsexual routes, perhaps from oral contact (kissing), particularly in Mediterranean and African countries. Transmission directly from a pregnant mother to her fetus appears to be rare, although very young children can become infected after birth, particularly in Africa. This is a common feature for some herpesviruses in that infection with these viruses is general and occurs at an early age in impoverished countries. KSHV can be transmitted during organ transplantation although this appears to be uncommon. Once exposed to KSHV, infection is probably life-long and the immune system of healthy adults keeps the virus in check at extremely low levels. The real problem with KSHV occurs when an infected person becomes immunosuppressed. This occurs among transplant patients and patients receiving chemotherapy. There is good evidence that if someone is infected with KSHV and becomes immunosuppressed, then their chances of developing clinically detectable disease due to KSHV is higher than for other common viral infections. Infection with KSHV is diagnosed by a blood test. It now seems likely that early in the AIDS epidemic there were actually two simultaneous virus epidemics: the well-recognized epidemic of HIV and a second 'silent' epidemic of KSHV. Only those persons who became immunosuppressed by HIV developed KS. KSHV is probably less transmissible than HIV, certainly this is true from blood products and sharing needles, and so only the fraction of persons developing AIDS were infected with KSHV and developed KS. KSHV-related disease can also occur in persons without obvious immunodeficiency, but this is rare and primarily occurs among elderly men. There has been a major epidemic of KS in some parts of Africa, especially Eastern and Southern Africa, concurrent with the African HIV pandemic. KS is now the most common tumor reported in several African cancer registries. While most African KS patients are also HIV infected, these areas also have the world's highest rates of this disease for non HIV-infected persons. There is no vaccine for KSHV. Existing therapy is Ganciclovir.

### Varicella-zoster virus (VZV)

Chicken pox is an infection that is caused by the Varicella-zoster virus (VZV). VZV spreads in nasal discharge and in fluid from inside the chicken pox blisters. Chicken pox is very contagious, and 90% of people who are not immune will catch it when they are exposed. Epidemics are most common in the late winter and early spring, and children between ages 5 and 9 account for half of all cases.

Normally, chicken pox is a mild illness, but it can cause serious complications, including pneumonia, encephalitis, and serious bacterial infections of chicken pox blisters. After causing an attack of chicken pox, VZV remains in the body. It lies dormant in nerve cells and may be reactivated later in life to cause shingles. Children (and others) who develop varicella and have weakened immune systems may be treated with Acyclovir.

### Adenoviruses

Adenoviruses are a frequent cause of acute upper respiratory tract infections. In addition, they also cause a number of other types of infection. They were first isolated in 1953 by investigators trying to establish cell-lines from adenoidal tissue of children removed during tonsillectomy and from military recruits with febrile illness. Certain types of Adenovirus are commonly associated with particular clinical syndromes: Acute Respiratory Illness, Pharyngitis, Conjunctivitis, Keratoconjunctivitis. Most Adenovirus infections involve either the respiratory or gastrointestinal tracts or the eye. Adenovirus infections are very common, most are asymptomatic. Most people have been infected with at least 1 type at age 15. Virus can be isolated from the majority of tonsils/adenoids surgically removed, indicating latent infections. It is not known how long the virus can persist in the body, or whether it is capable of reactivation after long periods, causing disease (it is hard to isolate this occult virus as it may be present in only a few cells). It is known that virus is reactivated during immunosuppression, e.g. in AIDS patients.

There is no therapy for Adenoviruses. Inactivated vaccines have been developed and are routinely used for military recruits in some countries (e.g. USA). This is because adolescents and others in close daily contact are at risk for epidemic spread of respiratory infections. In recent years, there has been considerable interest in developing Adenoviruses as defective vectors to carry and express foreign genes for therapeutic purposes. One reason for this is that the Adenovirus genome is relatively easily manipulated in vitro and the genes coupled to the MLP are efficiently expressed in large amounts.

### Respiratory syncytial virus (RSV)

Respiratory syncytial virus (RSV) is a major cause of respiratory illness in young children. RSV infection produces a variety of signs and symptoms involving different areas of the respiratory tract, from the nose to the lungs.

In children younger than age three, RSV can cause a lower respiratory tract illness like bronchiolitis or pneumonia and may lead to respiratory failure. In this case, symptoms may include high fever, severe cough, wheezing, abnormally rapid breathing, difficulty breathing, and a bluish color of the lips or fingernails caused by lowered levels of oxygen in the blood. In infants with severe RSV infection, there may be abnormal retractions of the muscles between the ribs, as the child struggles to draw breath into infected breathing passages. RSV infections occur all over the world, most often in epidemics that can last up to five months, from late fall through early spring. Since 1990, epidemics have typically begun sometime between late October and mid-December, and peaked during January and February. Each year during these epidemic periods, about 90,000 infants and young children are hospitalized with RSV infections - and about 4,500 die.

The highest rates of RSV illness occur in infants two to six months old, with a peak at age two to three months. RSV infection is often carried home by a school-aged child and passed onto a younger one, especially an infant. When RSV infects a day-care center, it is not unusual to see 100% of the children come down with an RSV infection. RSV commonly spreads through hospitals, too, infecting both patients and staff.

Prevention and treatment: RespiGam is a vaccine used to protect infants who are considered to be at high risk for severe illness if they are infected with RSV, such as those with chronic lung diseases. This intravenous preventive treatment takes several hours to administer and must be repeated monthly during RSV season. A similar vaccine, Synagis, has become available more recently. It has replaced RespiGam as the treatment of choice for most high-risk children because it's given as monthly intramuscular injections, making it more convenient and less time-consuming to administer. Ribavirin is the only drug available, with only moderate efficacy.

The object of the present invention is to provide new pharmaceutically active agents which can be used as substances for the prophylaxis and/or treatment of virally induced infections and diseases including opportunistic infections. This object is solved by the disclosure of the independent claim. Further advantageous features, aspects and details of the invention are evident from the dependent claims. The description, the examples and the figure of the present application are helpful to understand the invention.

### Description of the invention

Object of the present invention is to provide new pharmaceutically active agents together with methods for treating virally caused infections and diseases, including opportunistic infections.

This object is solved by the disclosure of the independent claim. Further advantageous features, aspects and details of the invention are evident from the dependent claims. The description, the examples and the figures of the present application are helpful to understand the invention.

Disclosed are compounds of the general formula (I): 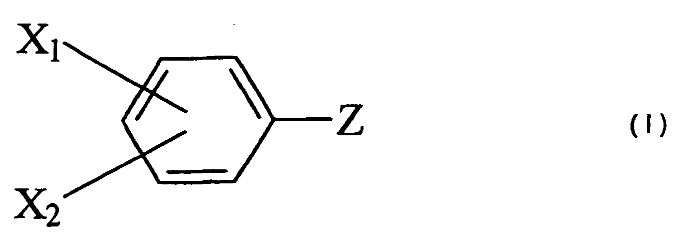 wherein:
X₁ and X'₁ are independently of each other -CHGhy or -C(CH₃)Ghy;
X₂ and X'₂ are independently of each other -H, -OCH₃, -CHGhy or -C(CH₃)Ghy;
Ghy represents a guanidino group: =N-NH-C(NH)NH₂;
Z is -A-(CH₂)ₙ-CH=CH-(CH₂)ₚ-A'-B, -A-(CH₂)ₙ-C₆H₄-(CH₂)ₚ-A'-B, -A-(CH₂)ₙ-C₅H₃N-(CH₂)ₚ-A'-B, -A-(CH₂)ₙ-CR'R"-(CH₂)ₚ-A'-B, -A-(CH₂)ₙ-CH=CH-(CH₂)ₚ-B, -A-(CH₂)ₙ-C₆H₄-(CH₂)ₚ-B, -A-(CH₂)ₙ-C₅H₃N-(CH₂)ₚ-B, -(CH₂)ₙB, -A-(CH₂)ₙ-CR'R"-(CH₂)ₚ-B, A-(CH₂)ₙ-B or -A-(CH₂)ₙ-A'-B;
R', R" are independently of each other -OH, -SH, -NH₂, methyl, ethyl or propyl;
A and A' are independently of each other -NH(CO)-, -NH-, -(CO)NH-, -NH(CO)NH- or -O-;
B represents 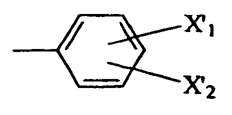
n and p are independently of each other integer of 0 to 10;
and pharmaceutically acceptable salts thereof under the proviso that A ≠ A'.

Compounds having the general formula (I) wherein A = A' are known from U.S. patents 5,599,984; 5,750,573; 5,753,684 as inhibitors of the uptake of arginine by macrophages and/or its conversion to urea. Therefore, these compounds can be used for treating arginine-dependent tumors and infections. Further, the suitability of these compounds in preventing the generation of nitric oxide (NO) by cells and to prevent NO-mediated inflammation and other responses is disclosed. WO98/20868 discloses a method for treating diseases and disorders involving T-cell activation and HIV infection using the p38 mitogen activated protein kinase (MAPK) signaling pathway as target for the intervention. It is suggested that guanylhydrazone compounds according to formula (I) act as MAPK inhibitors. U.S. 5,849,794 discloses the use of some compounds of the general formula (I) wherein A = A' for preventing and ameliorating cachexia, the clinical syndrome of poor nutritional status and bodily wasting associated with cancer and other chronic diseases, while U.S. 5,854,289 describes the use of said compounds for treating multiple sclerosis (autoimmune encephalomyelitis) and U.S. 6,143,728 discloses their use for treating a disorder or disease characterized by T cell activation.

Surprisingly, the guanylhydrazone compounds of the present invention exhibit excellent activity against a variety of different viruses.

Most preferred is the use of the inventive compound
N,N'-bis (3,5-diacetylphenyl) decane diamide tetrakis (amidinohydrazone), or a salt thereof.

Disclosed are compounds of the **general formula (II):** 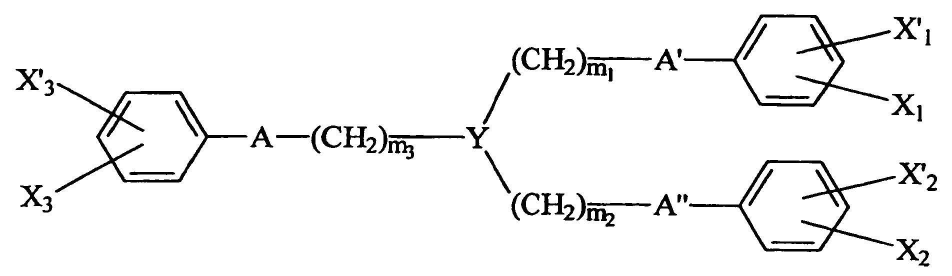 wherein:
X₁, X₂ and X₃ are independently of each other -CHGhy or -C(CH₃)Ghy;
X'₁, X'₂ and X'₃ are independently of each other -H, -CHGhy or -C(CH₃)Ghy;
Ghy represents a guanidino group: =N-NH-C(NH)NH₂;
A, A' and A" are independently of each other -NH(CO)-, -(CO)NH-, -NH(CO)NH-, -NH- or -O-;
Y is (C₆H₃), when m₁, m₂, m₃ = 0;
Y is N, when m₁, m₂, m₃ are independently of each other integer of 1 to 6;
and pharmaceutically acceptable salts thereof as pharmaceutically active agents for prophylaxis and/or treatment of virally induced infections and diseases, including opportunistic infections.

Compounds of the general formula (II) are known from U.S. 5,859,062 and 5,849,794 wherein the use of such compounds for treating inflammatory diseases mediated by TNF and a method for treatment of neoplastic diseases are disclosed. Furthermore, compounds of the general formulas (I) and (II) are described in U.S. 6,022,900 and 6,008,255 together with their use for treating whole body nitrogen loss associated with catabolic illness or for treating endotoxic shock in a subject.

Preferably is the use of a compound of general formula (II) wherein X₁ is in meta or para position to A' in the case that X'₁ represents hydrogen; and/or wherein X₂ is in meta or para position to A" in the case that X'₂ represents hydrogen; and/or X₃ is in meta or para position to A in the case that X'₃ represents hydrogen. If the residue X₁ and/or X₂ and/or X₃ is not hydrogen, the meta position of either X₁ and X'₁ in relation to A', and/or the meta position of X₂ and X'₂ in relation to A", and/or the meta position of X₃ and X'₃ in relation to A is/are preferred.

The compounds of the general formula (II) exhibit three of the characteristic structural elements as shown in the general formula (I), comprising a benzene core substituted with one or two residues characterized as X and one linker (characterized as Z) represented by an alkyl chain consisting of 1 to 6 methylene units connected to said benzene core via the structural element A as shown in formula (II).

Disclosed is the use of the aromatic guanylhydrazone compounds of the general formula (I) and/or (II) and/or pharmaceutically acceptable salts thereof as an inhibitor of deoxyhypusine synthase.

Disclosed is the use of a compound of the general formula (I) and/or (II) and/or pharmaceutically acceptable salts thereof as an inhibitor of nuclear transport or export in infectious diseases.

Surprisingly, it was found that the guanylhydrazone compounds of the general formula (I) and (II) exhibit excellent activity against various viruses and can therefore be used for prophylaxis and treatment of virally induced infections, including opportunistic infections, and also diseases associated with such infections.

The compounds of the present invention and/or pharmaceutically active salts thereof can be used for the manufacture of an agent for prophylaxis and/or treatment of diseases and infections, including opportunistic infections, caused by viruses. Examples for such viruses are retroviruses (lentiviruses and oncoretroviruses), adenoviruses, hepadnaviruses, herpesviruses, influenza viruses and paramyxoviruses. Viruses which integrate in the genome of a cell are retroviruses, hepadnaviruses, adenoviruses, herpesviruses and influenza viruses while paramyxoviruses do not integrate in the genome of a cell are.

The retroviruses may be selected from the group comprising lentiviruses and oncoretroviruses. Examples for lentiviruses are FIV, SIV, BIV, HIV-1, HIV-2, visna virus, caprine arthritis-encephalitis virus (CAEV), and equine infectious anemia virus (EIAV). Most preferably, the retrovirus represents the lentivirus HIV-1 or HIV-2. HTLV-I, HTLV-II and BLV belong to the oncoretroviruses. Furthermore, the excellent antiviral activity of the inventive guanylhydrazone compounds can perferably be used to treat retroviruses wherein the retrovirus is a T-cell tropic HIV strain or wherein the retrovirus is a macrophage-tropic HIV strain.

Paramyxoviruses comprise respiratory syncytial virus, parainfluenza viruses, mumps virus, and measles virus. More preferably, the paramyxovirus is respiratory syncytial virus. The herpesvirus family comprises the human herpesviruses 1 to 8 and different herpes viruses for various animal species as shown below in table 2:

**Table 2:**

| Members of the herpesvirus family | | | |
|---|---|---|---|
| **Subfamily** | **Genus** | **Human** | **Animal** |
| α-herpesvirus | simplex virus | human herpesvirus 1 | bovine herpesvirus 2 |
| | | (herpes simplex virus 1) | |
| | | human herpesvirus 2 (herpes simplex virus 2) | cercopithecine herpes-virus 1, (herpes B virus) |
| | varicella virus | human herpesvirus 3 | pseudorabiesvirus |
| | | (Varicella Zoster virus) | |
| | | | bovine herpesvirus 1 |
| | | | equine-abortion virus |
| β-herpesvirus | cytomegalovirus | human herpesvirus 5 | |
| | | (HCMV) | |
| | muromegalovirus | | murine herpesvirus 1 |
| | roseolovirus | human herpesvirus 6, | aotine herpesvirus 1, 3 |
| | | human herpesvirus 7 | |
| γ-herpesvirus | lymphocrytovirus | human herpesvirus 4 (Epstein-Barr virus) | cercopithecine herpes-virus 2 |
| | | | pongine herpesvirus 1 |
| | rhadinovirus | human herpesvirus 8 | ateline herpesvirus 2 |
| | | | saimirine herpesvirus 1 |

More preferably, the herpesvirus is selected from Herpes simplex virus I, Herpes simplex virus II, Varicella Zoster virus, Epstein-Barr virus, HCMV, or HHV8. The hepadnavirus are selected from HBV, Ground-Squirrel-Hepatitis virus (GSHV), or Woodchuck hepatitis virus (WHV).

The HIV is according to Gallo and Barre-Sinoussi the causative agent of the acquired immune deficiency syndrome (AIDS) (Barre-Sinoussi et al., 1983; Gallo et al., 1984). In humans, HIV infection leads to development of immune incompetence, opportunistic infections, neurological disorders like cognitive and motor impairment, neoplastic growth, and death. HIV infection is pandemic and HIV associated diseases represent a major, increasing world health problem. Considerable effort is being put into the design of effective therapeutics, but no curative anti-retroviral drugs against AIDS exist. Therefore, design and testing of effective, non-toxic, novel anti-retroviral drugs with novel modes of action are still needed.

The excellent antiviral activity of the aromatic guanylhydrazones of formula (I) allow their use for treating viruses resistant against the common antiviral drugs. Especially, the guanylhydrazone compounds represent a new class of anti-retroviral drugs.

The HIV is a complex retrovirus encoding in addition to the structural proteins gag, pol, and env several regulatory genes (Cullen 1991). Specifically, the trans-acting regulator of viral gene expression, called Rev, is crucially important for HIV replication (Feinberg et al., 1986). The HIV Rev protein is a nuclear phosphoprotein accumulating at steady state in the nucleoli and has the capacity to shuttle between the nuclear and cytoplasmic compartments (Meyer & Malim, 1994). Within the Rev protein distinct biological domains exist that are essential for its biological activity. The cis-acting target for Rev is a highly structured sequence element termed the Rev Response Element (RRE) located on HIV RNA (Malim et al., 1989). Upon binding to the HIV RNA as a monomer, Rev has to multimerize and subsequently interact with cellularly encoded cofactors (Malim et al., 1989) on its way to export the HIV RNA from the nucleus to the cytoplasm where protein translation occurs. To date, Rev is the most studied specific RNA export factor containing a leucine-rich nuclear export signal (NES) (Pollard &Malim, 1998). It was shown that the eukaryotic initiaton factor 5A (eIF-5A) directly and specifically binds the HIV Rev NES (Ruhl et al., 1993). Distinct eIF-5A mutants have been shown to inhibit nuclear export of Rev proteins and, thereby, HIV replication (Bevec et al., 1996; Junker et al., 1996). Moreover, anti eIF-5A antibodies specifically block the nucleocytoplasmic translocation of Rev (Schatz et al., 1998), indicating that eIF-5A is part of a specific nucleocytoplasmic export pathway utilized by HIV (Elfgang et al., 1999). In general, the translocation of mRNAs across the nuclear pore requires a protein carrier, which is part of a complex system located in the nuclear envelope. In this view, elF-5A would be a part of a transport system which is involved in the translocation of nuclear RNA to the cytoplasm. After transit through the nuclear pores and assembly of translation-competent ribosomes, the preferentially translocated mRNAs would be utilized in the process of protein synthesis. Therefore, small molecular weight chemical substances that interfere with the biological function of the elF-5A protein at the level of nuclear transport are potential antiviral compounds by interfering with nucleocytoplasmic translocation of viral mRNAs, especially in the field of HIV associated diseases. Surprisingly, it was found that the aromatic guanylhydrazone compounds of the present invention act as an inhibitor of elF-5A activation.

The elF-5A protein has an unique biochemical feature in that it is the only eukaryotic cellular protein known containing the unusual amino acid hypusine. Hypusine formation is a modification of the lysine residue at amino acid position 50 within the 154 amino acids containing elF-5A precursor molecule by a two-step posttranslational enzymatic reaction (Park et al., 1993). In the first step, the cellular enzyme deoxyhypusine synthase (DHS) catalyzes the transfer of an aminobutyl-moiety from spermidine to the lysine 50 within elF-5A via an enzyme-substrate intermediate formation at lysine 329 of the DHS to generate the elF-5A deoxyhypusine form (Wolff et al., 1997). This elF-5A deoxyhypusine intermediate is subsequently hydroxylated by the enzyme deoxyhypusine hydroxylase, resulting in biologically active, hypusine-containing elF-5A. Hypusine formation is essential for the biological activity of the elF-5A protein (Park et al., 1993). In addition, biologically active elF-5A protein is essential for nuclear export of Rev proteins and, thereby, HIV replication (Bevec et al., 1996; Junker et al., 1996), as well as for the function of the Human T-cell leukemia virus type I (HTLV-I) Rex protein which is itself crucial for HTLV-I replication (Katahira et al., 1995).

Therefore, an in vitro DHS screening assay was established (Bevec et al., 1996) in order to identify small molecular weight chemical substances as inhibitors of hypusine formation on the elF-5A protein with the goal to ultimately interfere with HIV and HTLV-I replication. In addition, such inhibitors may interfere with the replication of influenza viruses (targeting the biological activities of NS1, NS2, or M1 proteins), with the replication of herpes viruses (targeting the biological activities of the Epstein-Barr Virus SM protein and the Herpes simplex virus ICP27 protein), or with the replication of adenoviruses (targeting the biological activities of E1 B and E4orf6 proteins).

Surprisingly it was found that the aromatic guanylhydrazones and pharmaceutically acceptable salts thereof are potent inhibitors of elF-5A activation which exhibit their effect at pharmaceutically acceptable concentrations. Further, the aromatic guanylhydrazones are capable of inhibiting nuclear transport of RNA molecules. Without wishing to be bound to a theory, Applicant believes that particularly the RNA polymerase III-like export of mRNA molecules (mRNA molecules are usually believed to be exported from the nucleus to the cytoplasma by the RNA polymerase II-dependent export pathways) from the nucleus to the cytoplasm of a cell may be inhibited. The inhibition of the nuclear transport represents a novel target for prevention or treatment of viral diseases, particularly HIV and opportunistic infections associated therewith.

Employing the in vitro DHS screening assay (Bevec et al., 1996) small molecular weight compounds from the class of the inventive aromatic guanylhydrazones like N,N'-bis(3,5-diacetylphenyl)-decane diamide tetrakis(amidinohydrazone) (Compound 4: also known as N,N'-bis[3,5-bis[1-(aminoiminomethyl) hydrazono] ethyl] phenyl decane diamide), compound 1, compound 5 were discovered as potent inhibitors of the hypusine formation on the elF-5A protein in submicromolar and low micromolar concentration ranges, respectively. Testing of compounds of the general formula (I) in relevant HIV cellular systems revealed that these compounds are potent inhibitors of HIV replication in the submicromolar and low micromolar range. Thus another aspect of the present invention provides the use of the compounds as claimed as inhibitors of elF-5A activation and HIV replication.

Surprisingly, in relevant HIV cellular systems compound No. 4 was discovered as potent inhibitor of HIV replication in the submicromolar range on an aggressive T-cell-tropic HIV strain, on an aggressive macrophage-tropic HIV strain, on primary HIV isolates from patients, as well as on an aggressive multidrug-resistant HIV strain without apparent cellular toxicities at effective antiviral dosages. Hence, compound No. 4 is a potent nuclear export inhibitor interfering with HIV replication. The present invention therefore provides a genus of guanylhydrazone-substituted compounds that are useful as inhibitors of HIV-1, HIV-2, HTLV-I as well as for treating diseases associated with those viruses.

The compounds of the present invention act as inhibitors of deoxyhypusine synthase and also as inhibitors of elF-5A activation and HIV replication. Thus, a further aspect of the present invention provides a method for inhibiting deoxyhypusine synthase activity for the prevention or treatment of infectious diseases, particularly viral infections, comprising administering a subject in need thereof a pharmaceutically effective amount of at least one compound of the general formula (I) and/or (II) and/or pharmaceutically active salts thereof.

Since eIF-5A is involved in the nuclear transport, the compounds of the present invention act as modulators of nuclear transport, particularly nuclear transport of RNA molecules. More particularly, the compounds of the inventions may be used for the manufacture of an agent capable of inhibiting the export of RNA molecules from the nucleus to the cytoplasm of a cell, which may be an infected mammalian cell, e.g. a human, bovine, equine, feline, caprine or ovine cell. Preferably, the compounds of formula (I) are inhibitors of the export of viral RNA molecules from the nucleus to the cytoplasm. More preferably, the compounds inhibit the export of RNA molecules which are derived from viral genetic material integrated in the genome of a host cell. Furthermore, it is preferred that the compounds (I) and/or (II) are used as modulators of RNA polymerase III dependent export of RNA molecules. Thus, disclosed is the use of at least one aromatic guanylhydrazone compound of the general formula (I) and/or pharmaceutically active salts thereof for the manufacture of an agent capable of modulating RNA polymerase III-like export of RNA molecules from the nucleus to the cytoplasma of a cell. Preferably said cell is a human cell.

By identifying the cellular targets of the compounds of formula (I) and/or (II) an agent for the prevention or treatment of infectious diseases involving nuclear export, particularly export of heterologous RNA from the nucleus to the cytoplasm via elF-5A is provided. Particularly, the present invention provides an agent and a method for the prevention or treatment of infectious diseases caused by viruses integrating and not integrating in the genome of a cell. The virus may be selected from the retroviruses, particularly lentiviruses and oncoretroviruses (HTLV-BLV group), adenoviruses, hepadnaviruses, herpesviruses and influenza viruses. More particularly, the virus is a retrovirus which may be selected from lentiviruses such as HIV-1, HIV-2, FIV, bovine immunodeficiency virus (BIV), simian immunodeficiency viruses (SIVs), visna Maedi virus (VMV), caprine arthritis-encephalitis virus (CAEV), equine infectious anemia virus (EIAV), or oncoretroviruses such as human T-cell leukemia virus I (HTLV-1), human T-cell leukemia virus II (HTLV-II) and bovine leukemia virus (BLV). Most preferably, the retrovirus is HIV-1 or HIV-2 and the herpesvirus is Herpes simplex virus I, Herpes simplex virus II, Varicella Zoster virus, Epstein-Barr virus, HCMV, or HHV8.

The compounds of the present invention can also be used for the manufacture of an agent for prophylaxis and/or treatment of diseases and infections caused by viruses integrating or not integrating in the genome of a cell. Examples for viruses which do not integrate in the genome of a cell are paramyxoviruses. Paramyxoviruses comprise, for instance, parainfluenza viruses, mumps viruses, measles virus, and respiratory syncytial viruses. More preferably, the virus is respiratory syncytial virus.

The present invention discloses also a method wherein the inventive guanylhydrazone compounds are used to treat viruses which are resistant against most common antiviral drugs. Thereby, the aromatic guanylhydrazones and/or pharmaceutically acceptable salts thereof are administered in a dosage corresponding to an effective concentration in the range of 0.01-10 µM. More preferably, the inventive compounds of the present invention are administered in a dosage corresponding to an effective concentration in the range of 0.1-5 µM.

Surprisingly, it was found that the compounds of formula (I) and (II) are potent inhibitors of HIV replication in a submillimolar, especially submicromolar, range on aggressive T-cell tropic HIV strains, aggressive macrophage-tropic HIV strains, on primary HIV isolates from patents as well as on agressive multidrug-resistant HIV strains without apparent cellular toxicity at the effective antiviral dosage.

These findings suggest that the mentioned compound class of aromatic guanylhydrazones has advantages over the currently used drugs in the treatment of HIV diseases, e.g. the Highly Active Antiretroviral Therapy (HAART), consisting of Nucleoside Analog Reverse Transcriptase Inhibitors (NRTIs), Non-Nucleoside Reverse Transcriptase Inhibitors (NNRTIs) and Protease Inhibitors (PIs). This therapy is accompanied by quick emergence of resistant viral strains. In some patients, replication of resistant HIV-1 leads to emergence of more virulent variants of HIV-1, which are associated with an accelerated loss of CD4+ cells and confer a significantly increased risk of disease progression and death (D'Aquila et al., 1995). Resistance emerges as a consequence of the selective pressure of incompletely suppressive therapy and is determined by mutations in the HIV reverse transcriptase and protease genes. Primary mutations alter the binding of the drug to its target resulting in an increase in the amount of drug necessary to inhibit the enzyme. Secondary mutations increase resistance by improving the fitness of viruses carrying primary infections. In the case of NRTls, the mutation M148V is an example of a key mutation leading to high-level resistance. Additional positions of mutations include M41L, K70R, T215Y (Zidovudine), L74V (Didanosine), T69D (Zalcitabine), Y115F (Abacavir). Multidrug resistance conferring cross-resistance to the entire NRTI class is well recognized (Q151M and insertion mutation T69SSS). Cross-resistance is also extensive among the three currently used NNRTIs Efavirenz, Nevirapine and Delavirdine which makes them inactive against the virus expressing the K103N mutation in the reverse transcriptase gene. The K103N mutation acts by inhibiting formation of the drug-binding pocket. For this class of drugs, the "first shot" is most frequently the "only shot". Also, cross-resistance between the Pls is rather a rule than an exception (Indinavir and Ritonavir have almost identical resistance patterns - K20M, V32I, M36I, M46I, 154V, L63P. A71V, V82A/T/F, I84V, L90M - and Saquinavir-resistant strains are also cross-resistant to other Pls - L101, K20M, l84V, L90M) (Roberts et al., 1998). Even for the novel type of anti HIV drugs like T20, a 36 amino acid peptide derived from the HIV_{gp41} protein which inhibits fusion of the virus to the host cell, resistance has already been demonstrated in vitro (Kilby et al., 1998). In addition, patients on HAART face long-term side effects, including pancreatitis, peripheral neuropathies, hepatotoxicity, diabetes (Visnegarwala et al.,1997), or metabolic abnormalities in body fat redistribution (Gervasoni et al., 1999), as well as in the glucose metabolism and the cardiovascular field (Henry et al., 1998). For many patients, who experience the above mentioned drug toxicities, a structured treatment interruption is an unaviodable necessity ("Drug holidays"). However, during this time the suppressed virus may bounce back.

Due to the proposed mode of action as inhibitors of the biological function of the cellular eIF-5A protein which per se is a crucial cofactor for the function of the essential viral protein Rev for the translocation of essential HIV mRNAs from the nucleus to the cytoplasm, aromatic guanylhydrazones and substituted aromatic guanylhydrazones are very likely to decrease the resistance formation in the viruses for two main reasons: 1.) The primary target is a cellular protein; 2.) The viral Rev-RRE interaction is highly conserved. Mutations in the Rev activation domain (the interaction site of REV with elF-5A protein) are proven medical intervention strategies for inhibiting HIV replication (Bevec et al., 1992; Malim et al., 1992; Liu et al., 1994; Escaich et al., 1995; Woffendin et al., 1996; Bonyhadi et al., 1997; A molecular genetic intervention for AIDS-effects of a transdominant negative form of REV - General Clinical Research Center, University of Michigan, Ann Arbor, Center Watch; Clinical Trials Listing Service on Medscape, January 7, 2000).

Due to their reported ability to cross the blood-brain barrier in a rat animal model of permanent focal cortical ischemia (Meistrell et al., 1997) the compounds of formula (I) belong to the few anti-HIV substances (like Glaxo Wellcome's Abacavir, Lamivudine, Zidovudine or GW42867X, Bristol-Myers Squibb's Stavudine, or Roxane Laboratories' Nevirapine) which may also inhibit HIV replication in the central nervous system (CNS) for prevention or treatment of HIV-induced CNS disorders. Usually, the PI inhibitors are shown not to cross the blood-brain-barrier. Due to their mode of action, it may be reasonably expected that the compounds of general formula (I) and (II) will also inhibit the replication of other viruses as stated above.

In a related aspect to the studies disclosed herein, the present invention discloses to a method for treating virally induced diseases and infections, including opportunistic infections, in a mammal, including a human, which comprises administering to the mammal an amount of at least one compound of formula (I) and/or (II) and/or pharmaceutically acceptable salts thereof effective to treat virally induced infections and/or diseases. Preferably, said method is used for treating HIV-1 infections. Said virally induced diseases and infections may by cause by the viruses comprising retroviruses, adenoviruses, hepadnaviruses, herpesviruses, influenza viruses, and paramyxoviruses.

Hepadnaviruses may be selected from the group comprising orthohepadnaviruses and avihepadnaviruses. Examples for orthohepadnaviruses are HBV, Ground-Squirrel-Hepatitis virus (GSHV), Woodchuck Hepatitis virus (WHV). Most preferably, the hepadnavirus represents the human Hepatitis B virus (HBV).

Herpesviruses may be selected from the group comprising α-herpesviruses (Simplexvirus, Varicellavirus), β-herpesviruses (Cytomegalovirus also known as human herpesvirus 5, Muromegalovirus, Roseolovirus), or γ-herpesviruses (Lymphocryptovirus, Rhadinovirus). Examples for α-herpesviruses are Herpes simplex virus type 1 (human herpesvirus 1), Herpes simplex virus type 2 (human herpesvirus 2), Varicella Zoster virus (human herpesvirus 3). Examples for γ-herpesviruses are Epstein-Barr virus (human herpesvirus 4) or human herpesvirus type 8 (HHV8). More preferably, the herpesvirus is Herpes simplex virus type 1, or Varicella Zoster virus, or Epstein-Barr virus (EBV), or human cytomegalovirus (HCMV), or human herpesvirus 6, or human herpesvirus 7, or human herpesvirus type 8 (HHV8). Most preferably, the herpesvirus represents the α-herpesviruses Herpes simplex virus type 1, or Varicella Zoster virus, or the γ-herpesviruses Epstein-Barr virus, or Human Herpes virus type 8.

Paramyxoviruses may be selected from the group comprising paramyxovirinae or pneumovirinae. Most preferably, the paramyxovirus represents the respiratory syncytial virus (RSV).

In addition thereto, a method of inhibiting nuclear export for the prevention or treatment of infectious diseases, particularly viral infections comprising administering a subject in need thereof a pharmaceutically effective amount of at least one compound of the general formula (I) and/or pharmaceutically active salts thereof. Said infectious diseases, such as HIV-1 infections, or Hepatitis B virus infections, or Herpes-simplex-Virus 1 infections, or Epstein-Barr virus infections, or Human herpesvirus 8 infections, or Varicella-zoster virus infections, or Adenovirus infections, or Respiratory syncytial virus infections, are, for instance, diseases caused by viruses, especially lentiviruses or oncoretroviruses, hepadnaviruses, paramyxoviruses, adenoviruses, herpesviruses and influenza viruses. More preferably, said diseases are caused by the lentiviruses HIV-1 or HIV-2, or by the hepadnavirus Hepatitis B virus. The retrovirus may also be a T-cell tropic HIV strain, a monocyte-tropic HIV strain and most preferably a drug resistant virus strain. The HIV-1, or HIV-2 strain, or HBV strain may also be resistant against protease inhibitors and/or reverse transcriptase inhibitors. Thus, disclosed is the use of the guanylhydrazone compounds of the general formula (I) and/or (II) and a method for treatment of virally induced infections and diseases, including opportunistic infections, wherein the retrovirus is a HIV-1 or HIV-2 strain which is resistant against protease inhibitors and/or reverse transcriptase inhibitors.

In order to treat said virally induced infections and diseases associated thereto at least one compound of the general formula (I) and/or (II) and/or pharmaceutically effective salts thereof are administered to an individual in need according to the disclosed method in a dosage corresponding to an effective concentration in the range of 0.01-10 µM, more preferably in the range of 0.1-10 µM, most preferably 0.1-5 µM. Furthermore, the aromatic guanylhydrazone compounds may be administered directly or in combination with further therapeutic compounds, especially with further antiviral agents. A list of suitable antiviral agents is shown in table 3. In relation to the above statements, the use of the aromatic guanylhydrazone compound of the present invention and a method for the use of the inventive compounds and/or pharmaceutically active salts of said compounds is disclosed wherein at least one compound of the general formula (I) and/or (II) and/or pharmaceutically active salts thereof is administered in combination with further therapeutic compounds, especially with further antiviral agents. Said further antivaral agents may be selected from the drugs listed below in table 3.

Thus, the compounds described in the present invention can be used in a monotherapy directly or in form of pharmaceutically acceptable compositions in order to treat virally induced infections and/or diseases. Said diseases are preferably caused by or associated with HIV-1, HIV-2, HTLV-I, HBV. Furthermore, the inventive guanylhydrazone compounds can be used as inhibitors of HIV strains with tropism for monocytes and for strains with tropism for T cells. In addition, the compounds described in the present invention can be used in combination with other antiviral agents or drugs in order to combat HIV-1, HIV-2, HTLV-I, HBV as well as for treating diseases associated with those viruses.

The inventive compounds described in the present invention can be specifically combined with NRTIs and NNRTIs of HIV-1 and HIV-2 like: AZT (Zidovudine), 3TC (Lamivudine), ddl (Didanosine), ddC (Zalcitabine), ABC (Abacavir), d4T (Stavudine), FTC (2'deoxy-5-fluoro-3'thiacidin), Emivirine, EFV (Efavirenz), DLV (Delavirdine), NVP (Nevirapine), Adefovir dipivoxil, PMPA; with Pls Indinavir, Ritonavir, Saquinavir, Nelfinavir, Amprenavir, ABT378, BMS 232632, Tipranavir, L-756,423, DMP-450, AG-1776; with Hydroxycarbamid, Mycophenolat-mofetil; with fusion inhibitors T-20, T-1249; with CXCR4 antagonists like AMD 3100, T22, ALX40-4C, NSC 651016; with CCR5 antagonists like RANTES, APO-RANTES, NSC 651016; with Hydroxyurea; with Integrase inhibitors like antraquinones, quinalizarin, L-chicoric acid, dicaffeoylquinic acid; with Zinc Finger inhibitors like dithiane compounds; with immunomodulators like Interleukin-2, Interferon-alpha, Interferon-beta, GM-CSF, G-CSF; with therapeutical vaccine strategies including live, attenuated and replication incompetent virus; killed, inactivated virus; envelope subunit protein; core subunit protein; peptides; nucleic acids of the respective retroviruses, specifically of HIV-1 or HIV-2; with antiretroviral gene-therapy approaches like antisense or dominant-negative Rev mutants. This invention also relates to the combination of the inventive compounds described in the present invention with at least one of the above mentioned drugs. The inventive compounds of the general formula (I) and (II) can be used as inhibitors of HIV-1, HIV-2, HTLV-I, HBV as well as for treating diseases associated with those viruses, where the above mentioned antiviral agents and drugs, especially NRTIs, NNRTIs or PIs, caused viral resistances against said antiviral agents or drugs, respectively. The aromatic guanylhydrazone compounds of the present invention or pharmaceutically effective salts thereof are preferably administered in a dosage corresponding to an effective concentration in the range of 0.01-10 µM, more preferably in the range of 0.1-10 µM, and most preferably in the range of 0.1-5 µM.

As mentioned above, a preferred embodiment of the present invention relates to the use of the aromatic guanylhydrazone compounds claimed in combination with further antiviral agents. Table 3 represents a collection of suitable antiviral HIV agents which may be used in combination with at least one compound of the general formula (I) and/or (II) and/or pharmaceutically active salts thereof.

**Table 3:**

| Alphabetical List of Drugs for HIV (Experimental drugs are *italicized,* and approved drugs are in regular, non-italicized type) | |
|---|---|
| **Drug Name** | **Pharmaceutical Company** |
| *(*+*)-calanolide A* | *Sarawak Medichem* |
| 3TC, Epivir® brand lamivudine | Glaxo Wellcome |
| abacavir generic Ziagen™, ABC, or 1592U89 | Glaxo Wellcome |
| ABC, Ziagen™ brand abacavir, or 1592U89 | Glaxo Wellcome |
| ABT-378/r, or Kaletra™ brand lopinavir | Abbott Laboratories |
| *AG-1549**, S-1153, or capravirine (CPV)* | *Agouron Pharmaceuticals* |
| *AG1661, Remune™ brand HIV-1 Immunogen, or Salk vaccine* | *Agouron Pharmaceuticals* |
| Agenerase™ brand amprenavir (APV), or 141W94, VX-478 | Glaxo Wellcome |
| *aldesleukin generic Proleukin®, or Interleukin-2 (IL-2)* | *Chiron Corporation* |
| amprenavir generic Agenerase™, APV, 141W94, or VX-478 | Glaxo Wellcome |
| APV, Agenerase™ brand amprenavir, 141W94, or VX-478 | Glaxo Wellcome |
| AZT, Retrovir® brand zidovudine (ZDV) | Glaxo Wellcome |
| *BCH-10652**, or dOTC* | *BioChem Pharma* |
| *Bis(POC) PMPA*, *tenofovir disoproxil fumarate (TDF), or* GS-902 | *Gilead Sciences* |
| *BMS-232632* | *Bristol-Myers Squibb* |
| *capravirine* *(CPV),* AG-1549, *or S-1153* | *Agouron Pharmaceuticals* |
| *Coactinon®* *brand emivirine (EMV), or MKC-442* | *Triangle Pharmaceuticals* |
| Combivir® brand zidovudine + lamivudine, or AZT 3TC | + Glaxo Wellcome |
| *Coviracil*™ *brand emtricitabine , or FTC* | *Triangle Pharmaceuticals* |
| *CPV* *(capravirine), AG-1549, or S-1153* | *Agouron Pharmaceuticals* |
| Crixivan® brand indinavir (IDV), or MK-639 | Merck & Co. |
| d4T, Zerit® brand stavudine, or BMY-27857 | Bristol-Myers Squibb |
| *DAPD* | *Triangle Pharmaceuticals* |
| ddC, or Hivid® brand zalcitabine | Roche Laboratories |
| ddl, Videx® brand didanosine, or BMY-40900 | Bristol-Myers Squibb |
| delavirdine generic Rescriptor®, DLV, or 90152S/T | U-Agouron Pharmaceuticals |
| didanosine generic Videx®, ddl, or BMY-40900 | Bristol-Myers Squibb |
| DLV, Rescriptor® brand delavirdine, or U-90152S/T | Agouron Pharmaceuticals |
| *DMP-450* | *Triangle Pharmaceuticals* |
| *dOTC*, *or BCH-10652* | *BioChem Pharma* |
| Droxia® brand hydroxyurea (HU) | Bristol-Myers Squibb |
| efavirenz generic Sustiva™, EFV, or DMP-266 | DuPont Pharmaceuticals |
| EFV, Sustiva™ brand efavirenz, or DMP-266 | DuPont Pharmaceuticals |
| *emivirine* *generic Coactinon®, EMV, or MKC-442* | *Triangle Pharmaceuticals* |
| *emtricitabine* *generic Coviracil™, or FTC* | *Triangle Pharmaceuticals* |
| *EMV**, Coactinon® brand emivirine, or MKC-442* | *Triangle Pharmaceuticals* |
| Epivir® brand lamivudine, or 3TC | Glaxo Wellcome |
| epoetin alfa (erythropoietin) generic Procrit® | Ortho Biotech |
| erythropoietin (epoetin alfa) generic Procrit® | Ortho Biotech |
| Fortovase® brand saquinavir (Soft Gel Cap), or SQV (SGC) | Roche Laboratories |
| *FTC,* *or Coviracil™ brand emtricitabine* | *Triangle Pharmaceuticals* |
| *GS-902**, tenofovir disoproxil fumarate (TDF), or Bis(POC) PMPA* | *Gilead Sciences* |
| *GW-420867X* | *Glaxo Wellcome* |
| *GW-433908, or VX-175* | *Glaxo Wellcome* |
| | |
| *HIV-1 Immunogen generic Remune*™, *Salk vaccine, orAG1661* | *AgouronPharmaceuticals* |
| Hivid® brand zalcitabine, or ddC | Roche Laboratories |
| HU, or Droxia® brand hydroxyurea | Bristol-Myers Squibb |
| hydroxyurea generic Droxia®, or HU | Bristol-Myers Squibb |
| IDV, Crixivan® brand indinavir, or MK-639 | Merck & Co. |
| *IL-2* *(Interleukin-2), or Proleukin®* ^{*brand*} *aldesleukin* | *Chiron Corporation* |
| indinavir generic Crixivan®, IDV, or MK-639 | Merck & Co. |
| *Interfeukin-2* *(IL-2), or Proleukin®* ^{*brand*} *aldesleukin* | *Chiron Corporation* |
| Invirase® brand saquinavir (Hard Gel Cap), SQV (HGC), or Ro-31-8959 | Roche Laboratories |
| Kaletra™ brand lopinavir, or ABT-378/r | Abbott Laboratories |
| lamivudine generic Epivir®, or 3TC | Glaxo Wellcome |
| lopinavir generic Kaletra™, or ABT-378/r | Abbott Laboratories |
| *MKC-442* *Coactinon*® *brand emivirine (EMV)* | *Triangle Pharmaceuticals* |
| nelfinavir generic Viracept®, NFV, or AG-1343 | Agouron Pharmaceuticals |
| nevirapine generic Viramune®, NVP, or BI-RG-587 | Boehringer Ingelheim |
| NFV, Viracept® brand nelfinavir, or AG-1343 | Agouron Pharmaceuticals |
| Norvir® brand ritonavir (RTV), or ABT-538 | Abbott Laboratories |
| NVP, Viramune® brand nevirapine, or BI-RG-587 | Boehringer Ingelheim |
| *PNU-140690* *, or tipranavir* | *Boehringer Ingelheim* |
| Procrit® brand epoetin alfa (erythropoietin) | Ortho Biotech |
| *Proleukin*®^{*brand*} *aldesleukin, or Interleukin-2 (IL-2)* | *Chiron Corporation* |
| *Remune*™ *brand HIV-1 Immunogen, or Salk vaccine* | *Agouron Pharmaceuticals* |
| Rescriptor® brand delavirdine (DLV), or U-90152S/T | Agouron Pharmaceuticals |
| Retrovir® brand zidovudine (ZDV), or AZT | Glaxo Wellcome |
| ritonavir generic Norvir®, RTV, or ABT-538 | Abbott Laboratories |
| RTV, Norvir® brand ritonavir, or ABT-538 | Abbott Laboratories |
| *Salk vaccine, Remune*™ *brand HIV-1 Immunogen, or AG1661* | *Agouron Pharmaceuticals* |
| | |
| Saquinavir (Hard Gel Cap) generic Invirase®, SQV (HGC), or Ro-31-8959 | Roche Laboratories |
| Saquinavir (Soft Gel Cap) generic Fortovase®, or SQV (SGC) | Roche Laboratories |
| Serostim® brand somatropin | Serono Laboratories |
| Somatropin generic Serostim® | Serono Laboratories |
| SQV (HGC), Invirase® brand saquinavir (Hard Gel Cap), or Ro-31-8959 | Roche Laboratories |
| SQV (SGC), or Fortovase® brand saquinavir (Soft Gel Cap) | Roche Laboratories |
| stavudine generic Zerit®, d4T, or BMY-27857 | Bristol-Myers Squibb |
| Sustiva™ brand efavirenz (EFV), or DMP-266 | DuPont Pharmaceuticals |
| *T-20* | *Trimeris* |
| *TDF*, *tenofovir disoproxil fumarate, Bis(POC) PMPA, or GS-902* | *Gilead Sciences* |
| *Tenofovir disoproxil fumarate (TDF), Bis(POC) PMPA, or GS-902* | *Gilead Sciences* |
| *tipranavir**, or PNU-140690* | *Boehringer Ingelheim* |
| Trizivir™ brand abacavir + zidovudine + lamivudine (ABC + AZT + 3TC) | Glaxo Wellcome Glaxo Wellcome |
| Videx® brand didanosine, ddl, or BMY-40900 | Bristol-Myers Squibb |
| Videx® EC brand didanosine (ddl): delayed-release capsules | Bristol-Myers Squibb |
| Viracept® brand nelfinavir (NFV), or AG-1343 | Agouron Pharmaceuticals |
| Viramune® brand nevirapine (NVP), or BI-RG-587 | Boehringer Ingelheim |
| *VX-175**,* or GW-433908 | *Glaxo Wellcome* |
| zalcitabine generic Hivid®, or ddC | Roche Laboratories |
| ZDV, Retrovir® brand zidovudine, or AZT | Glaxo Wellcome |
| Zerit® brand stavudine, d4T, or BMY-27857 | Bristol-Myers Squibb |
| Ziagen™ brand abacavir (ABC), or 1592U89 | Glaxo Wellcome |
| zidovudine generic Retrovir®, AZT, or ZDV | Glaxo Wellcome |

### Opportunistic Infections (Ols)

As stated above, the inventive guanylhydrazone compounds of the present invention and/or pharmaceutically effective salts thereof or pharmaceutical compositions containing at least one inventive guanylhydrazone compound as an active ingredient are useful for prophylaxis and/or treatment of infections, including opportunistic infections, and diseases associated with these infections.

Kindly remember the previous section titled "HIV & AIDS":

AIDS (acquired immune deficiency syndrome) is a condition caused by HIV. This virus attacks the immune system, the body's "security force" that fights off infections. When the immune system breaks down "opportunistic infections" (Ols) take advantage of the body's weakened defenses. Therefore, to say that someone "died of AIDS" is not entirely accurate, since it is often the opportunistic infections that cause death.

One diagnostic parameter to detect AIDS is to look at the patient's CD4+ cell counts. Another way to detect AIDS is to look for Ols: if an HIV+ individual is diagnosed with an opportunistic infection this will be taken as indication for AIDS.

Thus, it is really important to develop new methods and to provide new pharmaceutically active compounds in order to prevent and treat said opportunistic infections.

Listed below are lessons about each of the major Ols & cancers that can occur during late-stage HIV disease:

### Bacterial Infections

- Mycobacterium Avium Complex (MAC, MAI)
- Salmonellosis
- Syphilis & Neurosyphilis
- Tuberculosis (TB)

### Malignancies (Cancers)

- Anal Dysplasia/Cancer
- Cervical Dysplasia/Cancer
- Kaposi's Sarcoma (KS)
- Lymphomas

### Viral Infections

- Cytomegalovirus (CMV)
- Hepatitis
- Herpes Simplex Virus (oral & genital herpes)
- Herpes Zoster Virus (shingles)
- Human Papiloma Virus (HPV, genital warts, anal/cervical dysplasia/cancer)
- Molluscum Contagiosum
- Oral Hairy Leukoplakia (OHL)
- Progressive Multifocal Leukoencephalopathy (PML)

### Fungal Infections

- Aspergillosis
- Candidiasis (thrush, yeast infection)
- Coccidioidomycosis
- Cryptococcal Meningitis
- Histoplasmosis

### Protozoal Infections

- Cryptosporidiosis
- Microsporidiosis
- Pneumocystis Carinii Pneumonia (PCP)
- Toxoplasmosis

### Neurological Conditions

- AIDS Dementia Complex
- Peripheral Neuropathy

### Other Conditions and Complications

- Apthous Ulcers
- Depression & Anxiety
- Fatigue and Anemia
- Nausea & Diarrhea
- Thrombocytopenia
- Wasting Syndrome & Lipodystrophy

The past ten years of study have produced clear evidence that individuals with HIV-1 infection are at risk for both primary and secondary neurological and neurobehavioral disorders. Primary disorders occur as a result of the influence of HIV-1 on the central nervous system (CNS); whereas, secondary disorders usually occur as a result of immune system deficiencies or treatment effects. Neurobehavioral disorders associated with HIV-1 infection may be complicated by preexisting or new onset psychological and emotional disorders. Clinical research in neurological, and particularly neurobehavioral disorders associated with HIV-1 infection has been complicated by methodological issues and controversies as well as ill-defined nosology.

In the following information from current research on HIV-1 related neurological disorders, diagnosis, and treatment with focus on the AIDS related neurobehavioral disorders and a brief review of secondary HIV-related neurological disorders are presented. The inventive guanylhydrazone compounds of the present invention are also useful for prophylaxis and/or treatment of the opportunistic diseases described below.

### HIV-1 Related Neurological and Neurobehavioral Disorders

### Primary Disorders

HIV-1 can directly invade the central nervous system (CNS). Viral infection in the CNS is most often seen in mono-nuclear microglial cells and multi-nucleated giant cells. Neuronal loss is usually secondary to the presence of HIV-1 in surrounding cells and not in the neurons themselves. The process by which neuronal death occurs is speculative, although proposed mechanisms include the production of cytokines that interfere with neuronal function, production of abnormal neurotransmitter metabolites that are neurotoxic, and the presence of certain viral fragments that interfere with neurotransmitter transmission.

HIV-1 associated CNS disorders include the neurobehavioral disorders, HIV associated minor cognitive disorder and HIV associated dementia, and the neurological disorder, HIV associated myelopathy.

### HIV Associated Minor Cognitive Disorder

HIV associated minor cognitive disorder may occur in patients who are otherwise asymptomatic or mildly symptomatic. The disorder is characterized by subcortical deficits of attention, information processing speed, learning and memory, and psychomotor skills. HIV associated minor cognitive disorder may be complicated by the presence of depression or anxiety, but is not caused by psychiatric problems.

Recent studies have shown that the presence of HIV associated minor cognitive disorder increases with worsening immune function. CD4 and CD8 lymphocyte counts, CD4/CD8 ratios, and the presence of beta-2 microglobulin (B2M) in both serum and cerebrospinal fluid have been shown to correlate with severity of HIV associated cognitive disorder. However, they are not pathognomonic for the disorder.

### HIV Associated Dementia

HIV associated dementia (HAD) is a progressive disorder that initially presents as apathy, inertia, cognitive slowing, memory loss, and social withdrawal. As it progresses, multiple cognitive functions become increasingly impaired. The terminal phases are characterized by global cognitive impairment, mutism, and severe psychomotor retardation. Unlike HIV associated minor cognitive disorder, HAD rarely develops prior to constitutional problems and usually does not develop prior to other AIDS defining illnesses. As with HIV associated minor cognitive disorder, thorough neuropsychological evaluation is recommended to assist in differential diagnosis and to identify the presence of any co-existing psychiatric disturbance.

### HIV Associated Myelopathy

HIV associated myelopathy is characterized by symptoms of weakness, incoordination, and/or urinary incontinence and signs of paresis, spasticity, and hyperreflexia. This condition affects approximately 20% of adult patient with AIDS, although evidence of myelopathy is found at autopsy in 50% of patients. This condition is often associated with co-existing cognitive dysfunction.

HIV-1 has been found in the spinal cord and CSF of patients with HIV associated myelopathy; however, it is uncertain whether HIV-1 is a direct pathogen. Other conditions such as vitamin B12 deficiencies cause similar disorders, particularly in the immune-compromised patient.

### Secondary Disorders

The immuno-compromised HIV patient is at risk for numerous peripheral and central nervous system disorders that are not caused directly by the HIV virus. Peripheral nerve disorders, including sensory neuropathy, inflammatory demyelinating polyneuropathies, mononeuropathies, and cranial neuropathies are found in HIV infected patients. The incidence of neuropathies increases with worsening immune system functioning and usually occur in the presence of other HIV related disorders; however, in rare instances, peripheral neuropathies may precede other HIV symptoms. HIV-1 associated myopathy is uncommon, but may present across all stages of disease.

Immune compromised patients are at risk for neurological opportunistic infections. Cryptococcal meningitis, toxoplasmosis, cytomegalovirus (CMV), and progressive multifocal leukoencephalopathy (PML) are seen in varying incidences among AIDS patients. CMV retinitis causes a hemorrhagic retinitis in up to 20% of AIDS patients. The treatment of choice is ganciclorvir, which has demonstrated a positive clinical response in approximately 80% of patients treated.

In addition to the opportunistic infections, AIDS patients are at risk for opportunistic CNS neoplasms, metabolic encephalopathies, cerebrovascular disease, and neurosyphilis.

### References HIV section:

*1*) *Science* 1983 May 20;220(4599):868-71; Isolation of a T-lymphotropic retrovirus from a patient at risk for acquired immune deficiency syndrome (AIDS); Barre-Sinoussi F, Chermann JC, Rey F, Nugeyre MT, Chamaret S, Gruest J, Dauguet C, Axler-Blin C, Vezinet-Brun F, Rouzioux C, Rozenbaum W, Montagnier L
*2.) Science* 1984 May 4;224(4648):500-3; Frequent detection and isolation of cytopathic retroviruses (HTLV-III) from patients with AIDS and at risk for AIDS; Gallo RC, Salahuddin SZ, Popovic M, Shearer GM, Kaplan M, Haynes BF, Palker TJ, Redfield R, Oleske J, Safai B, et al
*3.) FASEB J* 1991 Jul;5(10):2361-8; Regulation of HIV-1 gene expression; Cullen BR
*4.) Cell* 1986 Sep 12;46(6):807-17; HTLV-III expression and production involve complex regulation at the levels of splicing and translation of viral RNA; Feinberg MB, Jarrett RF, Aldovini A, Gallo RC, Wong-Staal F
*5.) Genes Dev* 1994 Jul 1;8(13):1538-47; The HIV-1 Rev trans-activator shuttles between the nucleus and the cytoplasm; Meyer BE, Malim MH
*6.) J Virol* 1989 Aug;63(8):3213-9; Functional characterization of a complex protein-DNA-binding domain located within the human immunodeficiency virus type 1 long terminal repeat leader region; Malim MH, Fenrick R, Ballard DW, Hauber J, Bohnlein E, Cullen BR
*7.) Annu Rev Microbiol* 1998;52:491-532 ; The HIV-1 Rev protein; Pollard VW, Malim MH
*8.) J Cell Biol* 1993 Dec;123(6 Pt 1):1309-20; Eukaryotic initiation factor 5A is a cellular target of the human immunodeficiency virus type 1 Rev activation domain mediating trans-activation; Ruhl M, Himmelspach M, Bahr GM, Hammerschmid F, Jaksche H, Wolff B, Aschauer H, Farrington GK, Probst H, Bevec D, et al
*9.) Science* 1996 Mar 29;271(5257):1858-60; Inhibition of HIV-1 replication in lymphocytes by mutants of the Rev cofactor elF-5A; Bevec D, Jaksche H, Oft M, Wohl T, Himmelspach M, Pacher A, Schebesta M, Koettnitz K, Dobrovnik M, Csonga R, Lottspeich F, Hauber J
*10.) Hum Gene Ther* 1996 Oct 1;7(15):1861-9; Intracellular expression of cellular elF-5A mutants inhibits HIV-1 replication in human T cells: a feasibility study; Junker U, Bevec D, Barske C, Kalfoglou C, Escaich S, Dobrovnik M, Hauber J, Bohnlein E
*11.) Proc Natl Acad Sci U S A* 1998 Feb 17;95(4):1607-12; Interaction of the HIV-1 rev cofactor eukaryotic initiation factor 5A with ribosomal protein L5; Schatz O, Oft M, Dascher C, Schebesta M, Rosorius O, Jaksche H, Dobrovnik M, Bevec D, Hauber J
*12.) Proc Natl Acad Sci U S A* 1999 May 25;96(11):6229-34; Evidence for specific nucleocytoplasmic transport pathways used by leucine-rich nuclear export signals; Elfgang C, Rosorius O, Hofer L, Jaksche H, Hauber J, Bevec D
*13.) Biofactors* 1993 May;4(2):95-104; Hypusine: its post-translational formation in eukaryotic initiation factor 5A and its potential role in cellular regulation; Park MH, Wolff EC, Folk JE
*14.) J Biol Chem* 1997 Jun 20;272(25):15865-71; Enzyme-substrate intermediate formation at lysine 329 of human deoxyhypusine synthase; Wolff EC, Folk JE, Park MH
*15.) J Virol* 1995 May;69(5):3125-33; Effects of translation initiation factor elF-5A on the functioning of human T-cell leukemia virus type I Rex and human immunodeficiency virus Rev inhibited trans dominantly by a Rex mutant deficient in RNA binding; Katahira J, Ishizaki T, Sakai H, Adachi A, Yamamoto K, Shida H
*16.) FEBS Lett* 1996 Jan 8;378(2):195-8; Molecular characterization of a cDNA encoding functional human deoxyhypusine synthase and chromosomal mapping of the corresponding gene locus; Bevec D, Kappel B, Jaksche H, Csonga R, Hauber J, Klier H, Steinkasserer A
*17.) AIDS* 1998 Mar 26;12(5):453-60; Resistance and cross-resistance with saquinavir and other HIV protease inhibitors: theory and practice; Roberts NA, Craig JC, Sheldon J
*18.) Nat Med* 1998 Nov;4(11):1302-7; Potent suppression of HIV-1 replication in humans by T-20, a peptide inhibitor of gp41-mediated virus entry; Kilby JM, Hopkins S, Venetta TM, DiMassimo B, Cloud GA, Lee JY, Alldredge L, Hunter E, Lambert D, Bolognesi D, Matthews T, Johnson MR, Nowak MA, Shaw GM, Saag MS
*19.) Ann Intem Med* 1997 Nov 15;127(10):947; Severe diabetes associated with protease inhibitor therapy; Visnegarwala F, Krause KL, Musher DM
*20.) AIDS* 1999 Mar 11;13(4):465-71; Redistribution of body fat in HIV-infected women undergoing combined antiretroviral therapy; Gervasoni C, Ridolfo AL, Trifiro G, Santambrogio S, Norbiato G, Musicco M, Clerici M, Galli M, Moroni M
*21.) Lancet* 1998 May 2;351(9112):1328; Severe premature coronary artery disease with protease inhibitors; Henry K, Melroe H, Huebsch J, Hermundson J, Levine C, Swensen L, Daley J
22.) *J Virol* 1999 Jul;73(7):5741-7; Selection and characterization of human immunodeficiency virus type 1 mutants that are resistant to inhibition by the transdominant negative RevM10 protein; Hamm TE, Rekosh D, Hammarskjold ML
*23.) Proc Natl Acad Sci U S A* 1992 Oct 15;89(20):9870-4; Inhibition of human immunodeficiency virus type 1 replication in human T cells by retroviral-mediated gene transfer of a dominant-negative Rev trans-activator; Bevec D, Dobrovnik M, Hauber J, Bohnlein E
*24.) J Exp Med* 1992 Oct 1;176(4):1197-201; Stable expression of transdominant Rev protein in human T cells inhibits human immunodeficiency virus replication; Malim MH, Freimuth WW, Liu J, Boyle TJ, Lyerly HK, Cullen BR, Nabel GJ
*25.) Gene Ther* 1994 Jan;1(1):32-7; Regulated expression of a dominant negative form of Rev improves resistance to HIV replication in T cells; Liu J, Woffendin C, Yang ZY, Nabel GJ
*26.) Hum Gene Ther* 1995 May;6(5):625-34; RevM10-mediated inhibition of HIV-1 replication in chronically infected T cells; Escaich S, Kalfoglou C, Plavec I, Kaushal S, Mosca JD, Bohnlein E
*27.) Proc Natl Acad Sci U S A* 1996 Apr 2;93(7):2889-94; Expression of a protective gene-prolongs survival of T cells in human immunodeficiency virus-infected patients; Woffendin C, Ranga U, Yang Z, Xu L, Nabel GJ
*28.) J Virol* 1997 Jun;71(6):4707-16; RevM10-expressing T cells derived in vivo from transduced human hematopoietic stem-progenitor cells inhibit human immunodeficiency virus replication; Bonyhadi ML, Moss K, Voytovich A, Auten J, Kalfoglou C, Plavec I, Forestell S, Su L, Bohnlein E, Kaneshima H
*29.) Shock* 1997 Nov;8(5):341-8; Tumor necrosis factor is a brain damaging cytokine in cerebral ischemia; Meistrell ME 3rd, Botchkina GI, Wang H, Di Santo E, Cockroft KM, Bloom O, Vishnubhakat JM, Ghezzi P, Tracey KJ
*30.) J Biol Chem* 1989 Jan 25;264(3):1578-83; Sequence determination and cDNA cloning of eukaryotic initiation factor 4D, the hypusine-containing protein; Smit-McBride Z, Dever TE, Hershey JW, Merrick WC

### Pharmaceutical Compositions

Disclosed are pharmaceutical compositions comprising at least one compound of the general formulas (I) and/or (II) and/or pharmaceutically acceptable salts thereof as an active ingredient and a pharmaceutically acceptable carrier, excipient, adjuvent and/or diluent.

The compounds of the general formulas (I) and/or (II) can also be administered in form of their pharmaceutically active salts optionally using substantially nontoxic pharmaceutically acceptable carrier, excipients, adjuvents or diluents. The medications of the present invention are prepared in a conventional solid or liquid carrier or diluent and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits. Other than oral adminisratable forms are also possible. The inventive guanylhydrazone compounds of the general formulas (I) and/or (II) or pharmaceutical preparations containing said compounds may be administed by any appropriate means, including but not limited to injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrally, intracutanly, intravaginally, intravasally, intranasally, intrabuccally, percutanly, sublingually, or any other means available within the pharmaceutical arts.

Within the disclosed methods the pharmaceutical compositions of the present invention, containing at least one aromatic guanylhydrazone compound of the general formula (I) or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.

Also included are solid form preparations which are intended to be converted, shortly before use, to liquid form preparations for either oral or parenteral administration. Such liquid forms include solutions, suspensions and emulsions.

The aromatic guanylhydrazone compounds of the present invention may also be deliverable transdermally. The transdermal compositions may take the form of creams, lotions, aerosols and/or emulsions and can be included in a transdermal patch of the matrix or reservoir type as a re conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophillic semi-solid matrix.

Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluent in the composition can range from about 5 to about 95% by weight of the total composition, preferably from about 25 to about 75%, more preferably from about 30 to about 60% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 2 to about 20% by weight of the composition, more preferably from about 5 to about 10% by weight.

Binders characterize substances that bind or "glue" powders together and make them cohesive by forming granules, thus serving as the "adhesive" in the formulation. Binders add cohesive strength already available in the diluent or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight.

Lubricant refers to a substance added to the dosage form to enable the tablet, granules, etc. after It has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.

Glidents are materials that prevent caking and improve the flow characteristics of granulations, so that flow is smooth and uniform. Suitable glidents include silicon dioxide and talc. The amount of glident in the composition can range from about 0.1% to about 5% by weight of the total composition, preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.1 to about 5% by weight of the composition, preferably from about 0.1 to about 1%.

Techniques for the formulation and administration of the aromatic guanylhydrazone compounds of the present invention may be found in "Remington's Pharmaceutical Sciences" Mack Publishing Co., Easton PA. A suitable composition comprising at least one compound of the invention may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of a compound of the general formula (I) refers to that amount of the compound that results in an at least partial inhibition of bacterial cell growth. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. More preferably, the dosage of the compound corresponds to an effective concentration in the range of 0.01-10µM. The actual amount of the composition administered will be dependent on the subject being treated, on the subject's weight, the severity of the affliction, the manner of administration and the judgement of the prescribing physician.

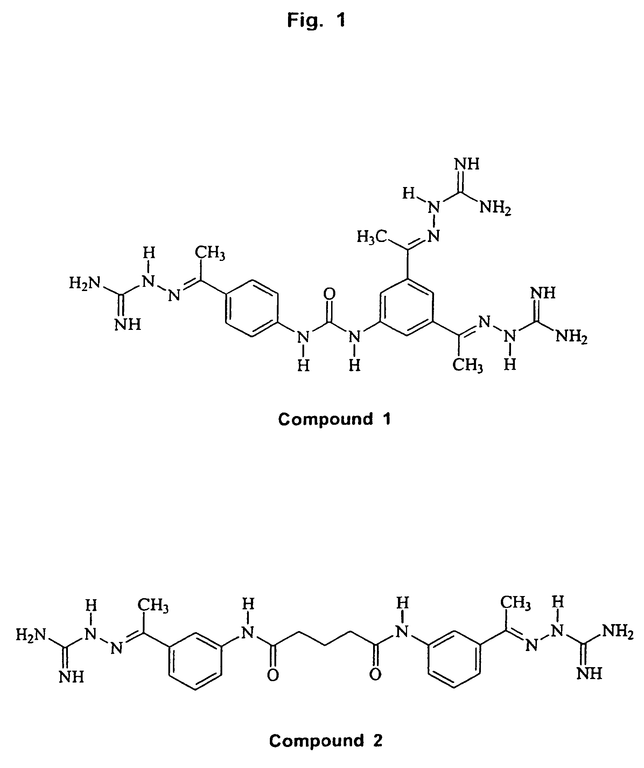

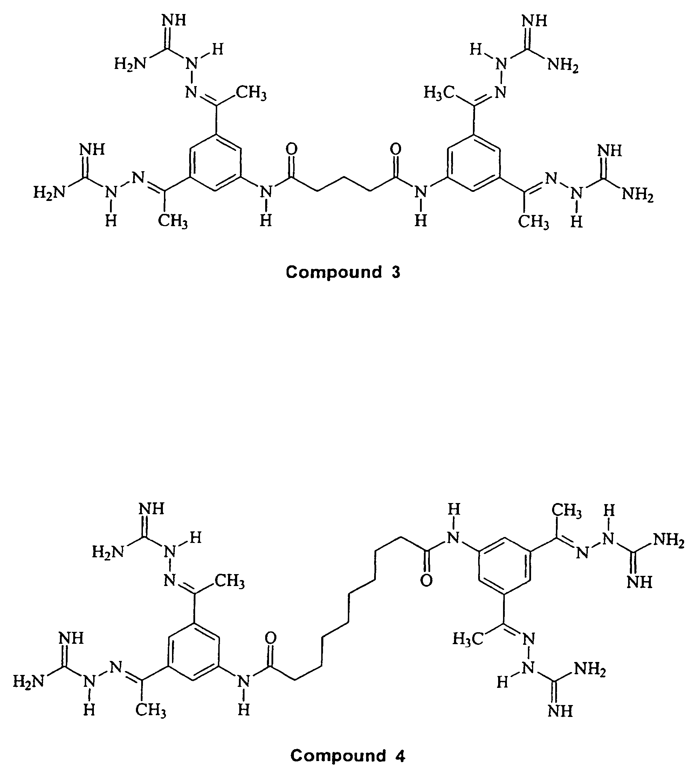

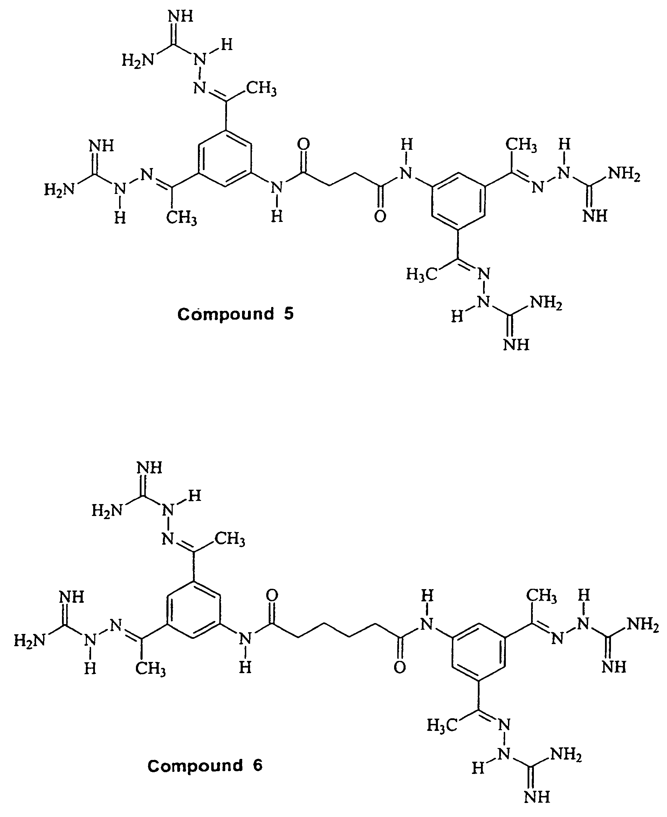

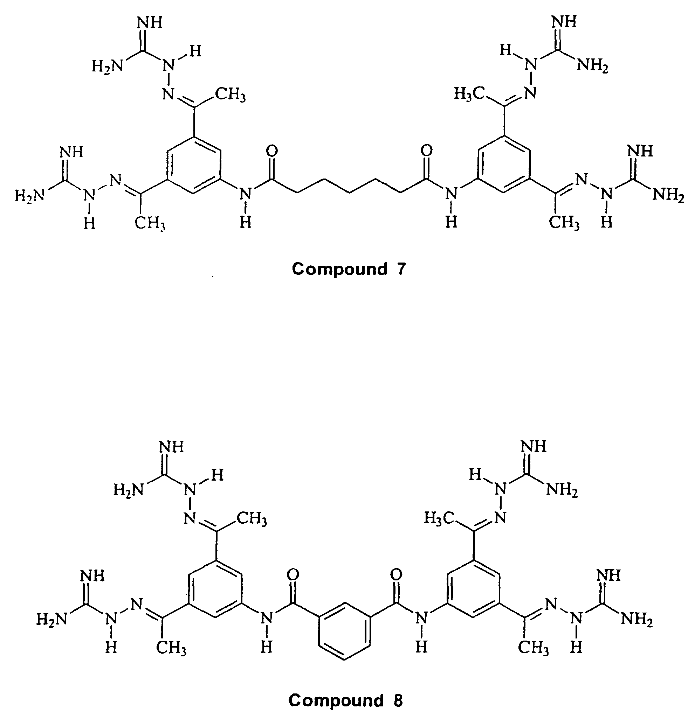

Fig. 1 shows the structures of several aromatic guanylhydrazone compounds of the general formula (I):

| | |
|---|---|
| Compound 1 | N-(4-acetylphenyl)-N'-(3,5-diacetylphenyl)urea tris (amidinohydrazone) |
| Compound 2 | N,N'-bis (3-acetylphenyl) pentane diamide bis (amidinohydrazone) |
| Compound 3 | N,N'-bis (3,5-diacetylphenyl) pentane diamide tetrakis (amidinohydrazone) |
| Compound 4 | N,N'-bis (3,5-diacetylphenyl) decane diamide tetrakis (amidinohydrazone) |
| Compound 5 | N,N'-bis (3,5-diacetylphenyl) butane diamide tetrakis (amidinohydrazone) |
| Compound 6 | N,N'-bis (3,5-diacetylphenyl) hexane diamide tetrakis (amidinohydrazone) |
| Compound 7 | N,N'-bis (3,5-diacetylphenyl) heptane diamide tetrakis (amidinohydrazone) |
| Compound 8 | N,N'-bis (3,5-diacetylphenyl) isophthalic acid diamide tetrakis (amidinohydrazone) |

### Examples

### Materials and Methods

**Cloning of the human elF-5A and DHS cDNA** was achieved using a premade liver cDNA library (Clontech) with polymerase chain reaction (PCR) primers specific for elF-5A (Smit-McBride et al., 1989), and DHS (Bevec et al., 1996) cDNAs harbouring additional BamHI and EcoRI restriction sites for positional cloning into protein expression vectors pGex (Amersham Pharmacia Biotech). After sequence determination, the plasmids were transformed into the E. coli strain BL21 (DE3). An overnight bacterial culture was diluted 1 : 10 in fresh LB-medium (supplemented with 100 µg/ml ampicillin) and grown for 1h at 37°C. Subsequently, IPTG was added (0.5 mM) and culture proceeded for a further 4h. The cells were pelleted and resuspended in 2 ml of PBS containing 1 mM DTT. After sonication (twice for 30s at a setting of 10W) the lysates was centrifuged for 2 min at 14,000 rpm in an Eppendorf centrifuge and the pellet dissolved in 1 ml of 8 M urea and dialysed overnight against 0.3 M glycine-NaOH (pH 9.0) / 0.1% Tween 20. The consistency of the fusion proteins was tested on a SDS-PAGE gel. After that, the GST-elF-5A fusion was cleaved with Factor Xa (Roche) due to the manufacturer's recommendation, whereas the GST-DHS fusion protein was tested for deoxyhypusine synthase activity. The standard enzymatic reaction mixture (total volume 200 µl) contained 5 µg elF-5A, 0.5 µCi [³H]spermidine (15 Ci/mmol), 1 mM NAD⁺, 1 mM DTT and various quantities of GST-DHS in 0.3 M glycine-NaOH buffer, pH 9.0, supplemented with 50 µg/ml BSA. The mixture was incubated at 37°C for 3h and stopped by adding 100 µl of 20 mM spermidine in PBS. The reaction mixture was passed over Sephadex G-25 gel filtration columns (PD-10 columns, Amersham Pharmacia Biotech) in order to separate the labeled elF-5A from excess spermidine. The incorporated radioactivity was measured in a liquid scintillation counter. The [³H]spermidine was obtained from DuPont-NEN; the unlabeled spermidine from Calbiochem, and NAD⁺ from Roche.

### HIV Experiments

### HIV detection

HIV replication was measured either by p24 HIV Antigen Capture Assay Kit (ELISA; HIVAG-1 Monoclonal kit B1A011; Abbott) following the manufacturer's recommendation, or by using the Quantiplex HIV-1 RNA 3.0 Assay (bDNA) system (Chiron Diagnostics). The latter assay is a signal amplification nucleic acid probe assay (employing a sandwich nucleic acid hybridization procedure) for direct quantitation of HIV RNA in human plasma. There, in brief, HIV-1 is first concentrated from plasma by centrifugation. After release of genomic HIV-1 RNA from the virions, the RNA is captured to a microwell by a set of specific, synthetic oligonucleotide capture probes. A set of target probes hybridize to both the viral RNA and the pre-amplifier probes. The capture probes, comprised of 17 individual capture extenders, and the target probes, comprised of 81 individual target extenders, bind to different regions of the *pol* gene of the viral RNA. The amplifier probe hybridizes to the pre-amplifier forming a branched DNA (bDNA) complex. Then, multiple copies of an alkaline phosphatase labeled probe are hybridized to this immobilized bDNA complex. Detection is achieved by incubating the entire complex with a chemoluminescent substrate. Light emission is directly proportional to the amount of HIV-1 RNA present in each sample, and results are recorded as relative light units by an appropriate analyzer.

### Cells and HIV strains

Jurkat cells (species: human T-cell leukemia; special characteristics: cells are permissive for growth of T-cell tropic HIV strains; source:ATCC Cat. No. TIB 152) or PM1 cells (species: clonal derivative of human HUT 78 cells; special characteristics: cells are permissive for growth of macrophage-tropic and T-cell tropic HIV strains; source: Dr. Marvin Reitz, courtesy of the NIH AIDS Research and Reference Reagent Program Cat.No. 3038) were stimulated with Phytohemagglutinin (PHA-P, Product No. L9132 from Sigma) in a concentration of 2 µg/ml (1.5x10⁶ cells/ml) and Hexadimethrine Bromide (Polybrene, Product No. H9268 from Sigma) in a concentration of 2 µg/ml (1.5 x 10⁶ cells/ml) in RPMI 1640 medium containing 10 % fetal calf serum (FCS, Pansystems GmbH) and antibiotics for 3 days.

For HIV-1 infection, 5x10⁷ cells were resuspended in 500 µl culture medium without test drugs and incubated in a 50 ml blue-cap-tube at 37°C for 5 hrs with HIV-1 high titer viral stocks. Jurkat cells were infected with the T-cell tropic strain HIV-1 NL4-3, PM1 cells with the macrophage tropic strain HIV-1 Ba-L (both from the NIH AIDS Research and Reference Reagent Program). After infection, cells were washed twice with PBS without Ca²⁺ and Mg²⁺ to avoid false positive p24 antigen determination. Cells were resuspended, and identical aliquots (1x10⁶/ml) of infected cells were further cultured in 10 ml medium with test drugs at various concentrations, or in medium with DMSO as control for calculation of the inhibition of virus replication [in %].

Culture medium was changed and cells were split at days 3 and 7 of the experiments. Viability of the cells (Trypan-blue staining), cell counts and p24 antigen levels were determined at days 3, 7 and 10 of the experiments. No significant differences in cell viability were observed (∼85-90 % living cells).

### Inhibition of T-cell-tropic and macrophage-tropic HIV-1, Results:

| PM1 cells de novo infected with macrophage-tropic HIV-1_{Ba-L} strain | | | | | |
|---|---|---|---|---|---|
| DMSO (solvent control) | | Compound No.4 0.5µM | | Compound No.4 1.0µM | |
| d10: | 0% | d10: | 82% | d10: | 73% |
| | | | | | |

| DMSO (solvent control) | | Compound No.5 1.0µM | | Compound No.5 5.0µM | |
|---|---|---|---|---|---|
| d7: | 0% | d7: | 48% | d7: | 97% |
| d10: | 0% | d10: | 35% | d10: | 86% |
| | | | | | |

| DMSO (solvent control) | | Compound No.1 1.0µM | | Compound No.1 5.0µM | |
|---|---|---|---|---|---|
| d7: | 0% | d7: | 23% | d7: | 72% |
| d10: | 0% | d10: | 0% | d10: | 51% |

| Jurkat cells de novo infected with T-cell-tropic HIV-1_{NL4/3} strain | | | | | |
|---|---|---|---|---|---|
| DMSO (solvent control) | | Compound No.4 0.5µM | | Compound No.4 1.0µM | |
| d10: | 0% | d10: | 31% | d10: | 85% |
| | | | | | |

| DMSO (solvent control) | | Compound No.5 1.0µM | | Compound No.5 5.0µM | |
|---|---|---|---|---|---|
| d7: | 0% | d7: | 26% | d7: | 70% |
| d10: | 0% | d10: | 0% | d10: | 16% |
| | | | | | |

| DMSO (solvent control) | | Compound No.1 1.0µM | | Compound No.1 5.0µM | |
|---|---|---|---|---|---|
| d7: | 0% | d7: | 15% | d7: | 52% |
| d10: | 0% | d10: | 5% | d10: | 31% |

At day 10 of infection, the HIV strains replicated vigorously in untreated cultures. Numbers describe percent inhibition of HIV replication as compared to control experiments.

Peripheral blood mononuclear cells (PBMCs) from HIV-1-infected patients were isolated - (after informal consent was given) - by Biocoll (Biochrom) gradient centrifugation. 1x10⁶/ml PBMCs were cultured (5 ml in a six well 32-mm plate) in presence of the test drugs or in DMSO (control diluent) in RPMI 1640 medium supplemented with 10% autologous serum and 2 mM L-Glutamine at 37°C and 5% CO₂.

The culture medium was changed every 3 days during the experiments. Once a week, each culture was split 1:1 and 2x10⁶ feeder-PBMCs (per well) and recombinant Interleukin 2 (IL-2) (10U / ml] (Roche) was added. Prior to addition to the cell cultures, the feeder-PBMCs, prepared from 4 healthy donors, were treated for 4 days with PHA-P and PB (2 µg/ml each per 2x10⁶ cells). At day 14, viability of the PBMCs (Trypan-blue staining), cell count and virus load (p24 antigen: Innotest HIV Antigen mAb, lnnogenetics N. V., Gent, Belgium, or bDNA: HIV-1 RNA 3.0 Assay, Bayer AG, Tarrytown, NY, USA) was determined. No significant differences in cell viability were observed (~75-80 % vital cells).

### Inhibition of clinical HIV-1 isolates, Results:

| HIV-infected PBMCs from patient 1 | | | | | |
|---|---|---|---|---|---|
| DMSO (solvent control) | | Compound No.4 0.5µM | | Compound No.4 1.0µM | |
| d14: | 0% | d14: | 91.6% | d14: | 90.7% |

| HIV-infected PBMCs from patient 2 | | | | | |
|---|---|---|---|---|---|
| DMSO (solvent control) | | Compound No.4 0.5uM | | Compound No.4 1.0µM | |
| d14: | 0% | d14: | 85.8% | d14: | 97.6% |

| HIV-infected PBMCs from patient 3 | | | | | |
|---|---|---|---|---|---|
| DMSO (solvent control) | | Compound No.4 0.5µM | | Compound No.4 1.0µM | |
| d14: | 0% | d14: | 91% | d14: | 90% |

At day 14 of cultivation, the virus replicated vigorously in all untreated cultures. Numbers describe percent inhibition of HIV-1 replication as compared to control experiments.

### HIV-1 Drug Wash-Out Experiments

PM1 cells were infected with HIV-1 Ba-L (as described above) and cultured for 12 days in the presence of Compound No. 4 at a concentration of 500 nM. Subsequently, the cells were split 1:1. One of these cultures was further incubated in 500 nM of the test drug for another 12 days. The other culture was washed with Medium and incubated for 12 days in DMSO (control).

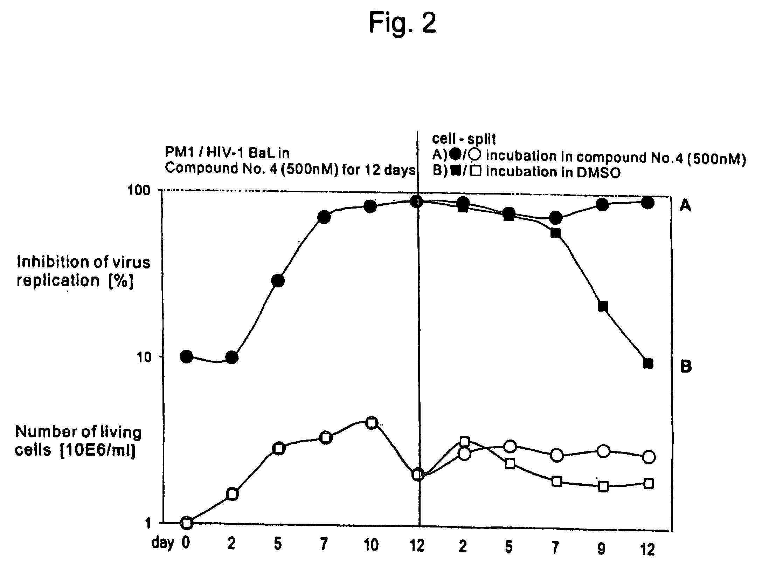

Culture medium was changed at day 2, 5, 7, 10 and 12 before the drug was removed and at day 2, 5, 7 and 9 after cell splitting. Viability of the cells, cell counts and p24 antigen levels were determined at the indicated days. Results from the wash-out-experiments with Compound No. 4 are shown in Figure 2.

### Inhibition of Antiviral Drug-Resistant HIV-1 Isolates

PM1 cells were infected with an "omni-drug-resistant HIV-1" (recombinant HIV-1 NL4-3 in which the Protease gene and the 5' part of the Reverse Transcriptase gene was replaced by the corresponding sequences from a omni-drug-resistant clinical isolate, resistant to all currently available anti-HIV drugs. Infected cells were incubated with Compound No. 4 at a concentration of 250, 500 and 1000 nM or DMSO (control). The culture medium was changed every third day. Cell viability (Trypan-blue staining), cell counts and p24 levels were determined at day 6 and 12.

| DMSO (solvent control) | | Compound No.4 0.25µM | | Compound No.4 0.5µM | | Compound No.4 1.0µM | |
|---|---|---|---|---|---|---|---|
| d6: | 0% | d6: | 15% | d6: | 30% | d6: | 58% |
| d12: | 0% | d12: | 64% | d12: | 95% | d12: | 97% |

At day 12 of infection, the virus replicated vigorously in untreated cultures. Numbers describe percent inhibition of HIV-1 replication as compared to control experiments.

In addition, various different "types" of multiple anti-retroviral drug resistant HIV-1 strains were also tested:

| **"Type" of Virus** | **Compound No. 4** **[µM]** | **[%] Inhibition of virus replication** |
|---|---|---|
| PI-resistant HIV-1 | 1.0 | 95 % |
| | 0.5 | 50 % |
| NNRTI-resistant HIV-1 | 1.0 | 82 % |
| | 0.5 | 71 % |
| NRTI + PI-resistant HIV-1 | 1.0 | 73% |
| | 0.5 | 68% |
| PI: Protease inhibitors | | |
| NNRTI: Non-Nucleoside Reverse Transcriptase Inhibitor | | |
| NRTI: Nucleoside Reverse Transcriptase Inhibitor | | |

### Long-Time Inhibition of a Clinical HIV-1 Isolate

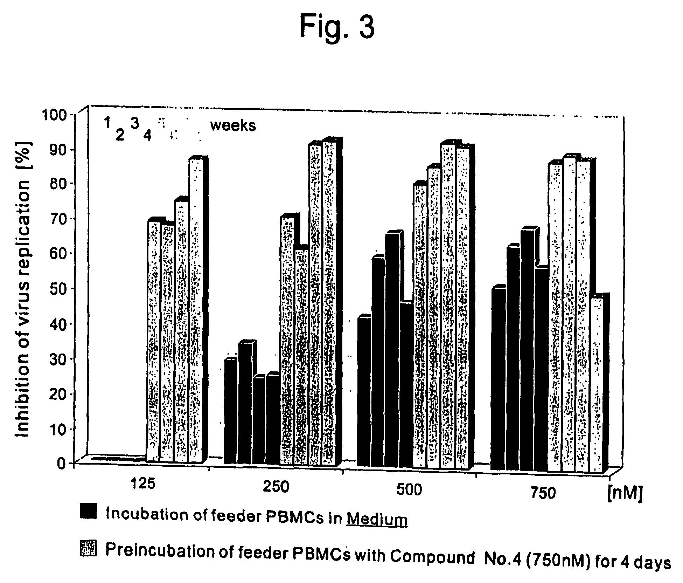

PBMCs from a HIV-1-infected patient were cultured in various concentrations of Compound No. 4 (125, 250, 500 and 750 nM) or DMSO (control). Culture medium was changed every 3 days during the experiments. Once a week, each culture was split 1:1 and feeder-PBMCs (feeder cells were prepared and stimulated with PHA-P and PB as outlined in Figure 3) and recombinant IL-2 [10 U / ml] (Roche) were added. Every week, cell viability (Trypan-blue staining), cell counts and p24 levels were determined. Results of long-time-inhibition of a clinical HIV-1 isolate are shown in Figure 3.

### Inhibition of cytoplasmic accumulation of incompletely-spliced viral mRNAs caused by compound No. 4

10⁸ PM1 cells were infected with HIV-1 Ba-L. Infected cells were washed twice in PBS, split 1:1 and cultured in either the presence of Compound No. 4 (800 nM) or DMSO (50 ml cultures each). RNAs were isolated at day 7 post-infection. Cytoplasmic (C) RNAs were prepared by lysis of the cells in NP-40 buffer (0.05% NP-40, 5 mM MgCl₂, 50 mM KCL, 10 mM Hepes-NaOH pH 7.6) as described previously (Greenberg and Ziff 1984 Nature 311:433-438). Lysates were cleared by centrifugation (Eppendorf table-top centrifuge; 14.000 rpm, 5 min., 4°C) and adjusted to an end concentration of 4 M Guanidine Isothiocyanate. This solution was loaded onto a 5.7 M CsCl cushion. Samples were subsequently spun using a Beckman TLX Ultracentrifuge (TLA 100 rotor) for 2.5 hrs at 100.000 rpm and 24°C. The RNA pellet was resolved in water, treated with phenol/chloroform/isoamylalcohol (25:24:1) and ethanol-precipitated. After centrifugation (Eppendorf table-top centrifuge; 14.000 rpm, 15 min., 4°C), RNA pellets were lyophilised and subsequently dissolved in water for further analysis. Total (T) RNAs were isolated by direct lysis of the cells in 4 M Guanidine Isothiocyanate and purified by centrifugation through a 5.7 M CsCl cushion as outlined above. 25 µg of each, cytoplasmic and total RNA samples, were fractionated by electrophoresis through 1.2% formaldehyde agarose gels, transfered onto Hybond N membranes (Amersham) and subjected to Northern analyses using HIV-1 U3-LTR- (5'-GGTGTGTAGTTCTGCCAATCAGGGAAGTAGCC-3') and U6 snRNA-specific (5'-GATTAGCATGGCCCCTGCGCAAGG-3') radiolabeled synthetic oligonucleotide probes as described previously (Valent et al. 1991 Proc. Natl. Acad. Sci. USA 88:3339-3342).

Detection of the exclusively nuclear U6 snRNA served as control for purity of cytoplasmic RNA samples. The HIV-1-specific probe detects three classes of viral mRNA: unspliced ~ 9kb-class, singly-spliced ~ 4 kb-class and multiply-spliced ~ 2kb-class. Importantly, cytoplasmic accumulation of the ~ 9kb-class and ~ 4 kb-class of viral RNA species is subject to HIV-Rev protein regulation.

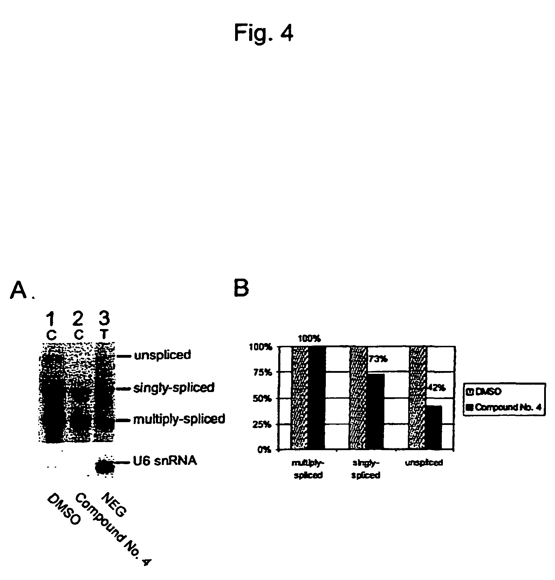

Intensities of specific bands were evaluated by phospho-imaging. To quantitatively compare the ~ 9kb-class and ~ 4 kb-class of cytoplasmic HIV-1 mRNA, the intensity-values were corrected due to slightly different RNA loads. For this, the intensities of the ~ 2 kb-class of HIV-1 mRNA (which accumulates in the cytoplasm in a HIV-Rev-independent fashion and appears to be unaffected by Compound 4 were equalised and the values for the unspliced and singly-spliced RNA species (~ 9kb-class and ~ 4 kb-class, respectively) were adjusted. Results of the inhibition of cytoplasmic accumulation of incompletely-spliced viral mRNAs caused by compound No. 4 are shown in Figure 4. Figure 4A shows HIV Ba-L. infected PM 1 cells. Figure 4B depicts the quantification of the blot shown in Figure 4A.

### Inhibition of the Hypusine-formation on elF-5A in living cells by the compound No. 4

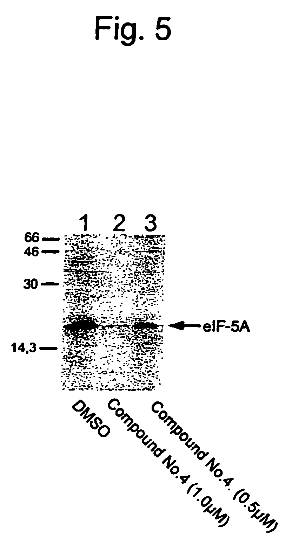

10⁷ Jurkat cells were metabolically labeled for 12 hours using 50 µCi [1,4-14C] Putrescine Dihydrochloride (108 mCi/mmol; Amersham) in the presence of 0.5 µM or 1.0 µM of compound No. 4 or DMSO (control). Cells were pelleted, washed twice in PBS and lysed in RIPA-buffer (0.1% SDS, 1% Triton X-100, 1% Sodium-Deoxycholate, 150 mM NaCl, 0.25 mM PMSF, 1mM EDTA, 10 mM Tris pH 7.4). Protein concentrations were determined (Bio-Rad protein assay) and equal protein amounts of the various radiolabeled cellular extracts were subjected to elF-5A-specific immunoprecipitation analyses using rabbit polyclonal anti-elF-5A antibody (Schatz et al. 1998 Proc. Natl. Acad. Sci. USA 95: 1607-1612) and analyzed by 14% SDS-PAGE and autoradiography. Results of Inhibition of the Hypusine-formation on elF-5A in living cells by the compound No. 4 are shown in Figure 5.

### Effects of compound No. 4 on apoptosis and cell-cycle progression

### Apoptosis-assay:

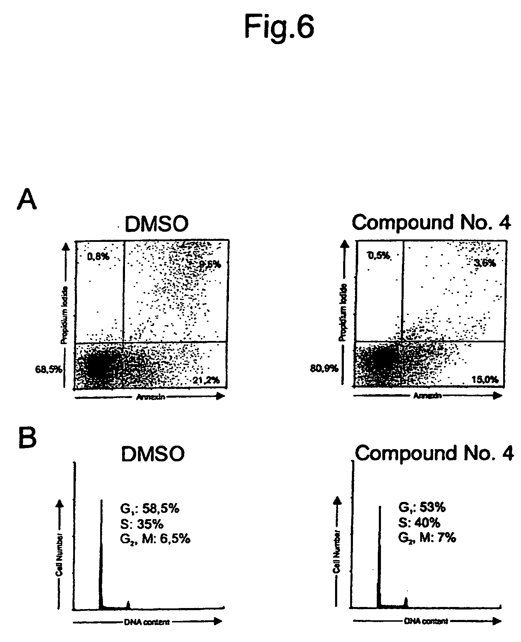

PBMCs from a healthy donor were cultured for 7 days in the presence of 500 nM of the Compound No. 4 or DMSO (control). Subsequently, cells were washed in PBS and assayed by FACS (FACSCalibur, Becton Dickinson) using a commercially available FITC-coupled annexin V apoptosis-kit according to the manufacturer's protocol (Bender Medsystems # BMS306FI). Results of the effects of compound No. 4 on apoptosis are shown in Figure 6A.

### Cell cycle-analysis:

Jurkat T-cells were cultured for 7 days in the presence of 500 nM of the Compound No. 4 or DMSO (control). Cell cycle analysis was recorded on a FACSCalibur device (Becton Dickinson) using a commercially available propidiumiodide-based DNA staining kit (CycleTest™ Plus; Becton Dickinson) according to the manufacturer's specifications. Results of the effects of compound No. 4 on cell-cycle progression are shown in Figure 6B.

### HBV Experiments

Human HepG2-2.2.15 cells (ATCC Cat. No. CRL-11997), chronically infected with HBV, are plated in 96-well microtiter plates in DMEM medium supplemented with fetal bovine serum. After incubation at 37°C in a humidified, 5% CO₂ environment for 16-24 hours, the monolayer of HepG2-2.2.15 cells is washed and the medium is replaced with complete medium containing various concentrations of test compound. Every three days during the experiments, the culture medium is replaced with fresh medium containing the appropriately diluted test compound.

Six days following the initial administration of the test compound, the cell culture supernatant is collected and clarified by centrifugation. The cell viability is evaluated by a dye uptake procedure (Promega's Cell Titer Aqueous One Solution). Virion-associated HBV DNA present in the tissue culture supernatant is amplified employing PCR technology using primer pairs derived from the HBV strain ayw. Subsequently, the PCR-amplified HBV DNA is detected in real-time by monitoring increases in fluorescence signals that result from exonucleolytic degradation of a quenched fluorescent probe molecule following hybridization of the probe to the amplified HBV DNA (TaqMan; Perkin-Elmer).
The same type of experiments was additionally performed with a mutant, Lamivudine-resistant HBV strain, which was transfected into HepG2 cells.

### Results from HBV experiments:

**Chronically HBV-infected HepG2 2.2.15 cells were treated with Compound No. 4**

| Compound | IC₅₀ (µm) Virion-DNA | IC₅₀ (µm) Intracellular Viral DNA | TC₅₀ (µm) | TI₅₀ Virion-DNA | TI₅₀ Intracellular Viral DNA | Comments |
|---|---|---|---|---|---|---|
| No.4 | 3.19 | 4.96 | >20 | >6.27 | >4.03 | Active |

Effect of Compound No. 4 on transfected, Lamivudine-resistant, mutant HBV production in HepG2 cells:

| Compound | IC₅₀ (µm) Virion-DNA | TC₅₀ (µm) | TI₅₀ Virion-DNA | Comments |
|---|---|---|---|---|
| No.4 | 2.78 | 24.7 | 8.7 | Active |

The minimum inhibitory drug concentration which reduces the viral replication by 50% (IC₅₀) and the minimum drug concentration which inhibits cell growth by 50% (TC₅₀) are calculated by using a regression analysis program for semilog curve fitting. A therapeutic (selectivity) index (TI) for compound No. 4 is determined by dividing the TC₅₀ by the IC₅₀.

### Herpes simplex virus type 1 experiments:

A virus-induced cytopathic effects (CPE)-inhibition assay procedure using Promega's Cell Titer Aqueous One Solution (MTS, metabolic dye) is employed to evaluate compounds for antiviral activity against herpes simplex virus type 1 (strain HF) in Vero cells (African green monkey cells, ATCC Cat. No. CCL-81). Vero cells are pregrown in 96-well tissue culture plates using Dulbecco's Modified Eagle's Medium (DMEM) supplemented with 10% heat-inactivated fetal bovine serum (FBS), L-glutamine, penicillin, and streptomycin. To each of the replicate cell cultures is added 50 µl of the test drug solution and 50 µl of virus suspension. Cell controls containing medium alone, virus-infected controls containing medium and virus, drug cytotoxicity controls containing medium and each drug concentration, reagent controls containing culture medium only (no cells), and drug colorimetric controls containing drug and medium (no cells) are run simultaneously with the test samples. The plates are incubated at 37°C in a humidified atmosphere containing 5% CO₂ until maximum CPE is observed in the untreated virus control cultures (Day 5). CPE inhibition is determined by a dye (MTS) uptake procedure. This method measures cell viability and is based on the reduction of the tetrazolium MTS by mitochondrial enzymes of viable host cells to MTS formazan. MTS (10 µl) is added to each of the plate wells. The plates are incubated at 37°C for 4 hours. The purple color of the MTS formazan is then measured spectrophotometrically at 490/650 nm. The optical density (OD) value of each culture is a function of the amount of formazan produced which is proportional to the number of viable cells. A computer program is utilized to calculate the percent of CPE reduction of the virus-infected wells and the % cell viability of uninfected drug control wells.

### Results from Herpes simplex virus type 1 experiments:

**HSV-infected Vero cells were treated with Compound No. 4**

| Compound | IC₅₀ (µM) | TC₅₀ (µM) | TI | Comments |
|---|---|---|---|---|
| No.4 | 1.56 | 20.58 | 13.19 | Active |

The minimum inhibitory drug concentration which reduces the CPE by 50% (IC₅₀) and the minimum drug concentration which inhibits cell growth by 50% (TC₅₀) are calculated by using a regression analysis program for semilog curve fitting. A therapeutic (selectivity) index (TI) for compound No. 4 is determined by dividing the TC₅₀ by the IC₅₀.

### Epstein-Barr virus experiments:

Human P3HR-1 cells are latently infected with EBV. Culturing these cells in the presence of phorbol ester induces a lytic infection cycle, resulting in stimulation of viral replication within the cells. Compound efficacy in this assay is determined using TaqMan PCR technology, and compound toxicity is measured by addition of Promega's Cell Titer Aqueous One Solution (metabolic dye).

### Assay Set-Up

1. P3HR-1 cells are counted by the trypan blue dye exclusion method, and the cell count/ml is determined. Cells are pelleted by centrifugation and washed once with PBS to minimize the basal level of virus released from untreated cells which spontaneously enter the lytic cycle and release virus.
2. Cells are resuspended in fresh media and adjusted to the appropriate cell density. Cells are then plated in the interior wells of 96-well round-bottom microtiter plates in a volume of 50 µl per well.
3. The phorbol ester TPA is added to appropriate wells in a volume of 50 µl per well to yield a final concentration of 15 ng/ml. Medium is added in place of TPA in three wells to serve as a negative control.
4. Test compounds are diluted in DMSO to a stock concentration which is 400x the desired high test concentration. These compounds are further diluted in complete medium in 1.2 ml titertubes to yield a solution which is 2x the desired high test concentration. Serial half-log dilutions of this solution are performed in complete medium to yield a total of 6 test concentrations for each test compound. Compound dilutions are added to appropriate wells of the microtiter plate in a volume of 100 µl per well. Medium containing no test compound is added to the virus control and cell control wells. In addition, 200 µl of medium containing no test compound is added to the exterior wells of the plate.
5. Plates are incubated at 37°C in a humidified CO₂ incubator until day 4 post drug addition.
6. Duplicate plates are set up plating media in place of TPA for evaluation of compound toxicity.

### Determination of Compound Efficacy and Toxicity

1. Efficacy plates are removed from the incubator, freeze thawed twice and cell lysate samples (100 µl) are removed from each well and transferred into 96-well storage plates.
2. Pronase is added to the supernatant samples to a final concentration of 0.75 mg/ml. Samples are incubated at 37°C for 30 minutes. Samples are then treated with 1 Unit of DNase per well and incubated at 37°C for 60 minutes. Encapsidated DNA from intact virions is not affected by this treatment. DNase is inactivated by heating samples to 95°C for 15 minutes.
3. PCR reaction mixtures are prepared from reagents provided in the PE Applied Biosystems TaqMan PCR Reagent Kit according to the manufacturer's directions. Total reaction volumes are 50 µl each. Master reaction mix is dispensed into optical PCR tubes in a volume of 47 µl. Samples (3 µl) are added to the reaction mix and mixed thoroughly.
4. Reaction plates are loaded into a PE Applied Biosystems 7700 Sequence Detector, and a run cycle is initiated using the manufacturer's recommended PCR conditions.
5. A standard curve prepared from known copy numbers of DNA isolated from P3HR-1 cells and amplified is run with each plate in order to quantitate the DNA copy number in each original sample. The efficacy of the compound is determined by comparing DNA copy numbers from test wells with those of control wells.
6. Twenty micorliters of Cell Titer Aqueous One Solution (Promega) is added to each well of the toxicity plates. Plates are incubated at 37°C in a humidified CO₂ incubator for 4 hours or until sufficient color development has occurred. Plates are read on a VMax microtiter plate reader at a wavelength of 490/650 nm. Toxicity of the test compound is determined by comparing the optical density of test wells with that of control wells.

### Results form Epstein-Barr virus experiments:

**EBV-infected P3HR1 cells were trated with Compound No. 4**

| Compound | IC₅₀ (µm) | TC₅₀ (µm) | TI | Comments |
|---|---|---|---|---|
| No.4 | 2.3 µM | 21.8 µM | 9,48 | Significant Activity |

The minimum inhibitory drug concentration which reduces the viral replication by 50% (IC₅₀) and the minimum drug concentration which inhibits cell growth by 50% (TC₅₀) are calculated by using a regression analysis program for semilog curve fitting. A therapeutic (selectivity) index (TI) for compound No. 4 is determined by dividing the TC₅₀ by the IC₅₀.

### Human Herpesvirus 8 experiments:

The human BCBL-1 cell line is available through the AIDS Reference and Reagent Program (Cat.No. 3233). This cell line was derived from a body cavity-based lymphoma latently infected with HHV-8. Culturing these cells in the presence of phorbol ester induces a lytic infection cycle, resulting in the release of viral particles into the medium. Compound efficacy in this proposed assay is determined using TaqMan PCR technology, and compound toxicity is measured by addition of Promega's Cell Titer Aqueous One Solution (metabolic dye).

### Assay Set-Up

7. BCBL-1 cells will be counted by the trypan blue dye exclusion method, and the cell count/ml will be determined. Cells will be pelleted by centrifugation and washed once with PBS to minimize the basal level of virus released from untreated cells which spontaneously enter the lytic cycle and release virus.
8. Cells will be resuspended in fresh media and adjusted to the appropriate cell density. Cells will then be plated in the interior wells of 96-well round-bottom microtiter plates in a volume of 50 µl per well.
9. The phorbol ester TPA will be added to appropriate wells in a volume of 50 µl per well to yield a final concentration of 15 ng/ml. Medium will be added in place of TPA in three wells to serve as a negative control.
10. Test compounds will be diluted in DMSO to a stock concentration which is 400x the desired high test concentration. These compounds will be further diluted in complete medium in 1.2 ml titertubes to yield a solution which is 2x the desired high test concentration. Serial half-log dilutions of this solution will be performed in complete medium to yield a total of 6 test concentrations for each test compound. Compound dilutions will be added to appropriate wells of the microtiter plate in a volume of 100 µl per well. Medium containing no test compound will be added to the virus control and cell control wells. In addition, 200 µl of medium containing no test compound will be added to the exterior wells of the plate.
11. Plates will be incubated at 37°C in a humidified CO₂ incubator until day 4 post drug addition.
12. Duplicate plates will be set up plating media in place of TPA for evaluation of compound toxicity.

### Determination of Compound Efficacy and Toxicity

7. Efficacy plates will be removed from the incubator, and supernatant samples (100 µl) will be removed from each well and transferred into 96-well storage plates.
8. Pronase will be added to the supernatant samples to a final concentration of 0.75 mg/ml. Samples will be incubated at 37°C for 30 minutes. Supernatant samples will then be treated with 1 Unit of DNase per well and incubated at 37°C for 60 minutes to degrade DNA which has been released by dead cells. Encapsidated DNA from intact virions will not be affected by this treatment. DNase is inactivated by heating samples to 95°C for 15 minutes.
9. PCR reaction mixtures well be prepared from reagents provided in the PE Applied Biosystems TaqMan PCR Reagent Kit according to the manufacturer's directions. Total reaction volumes will be 50 µl. Master reaction mix will be dispensed into optical PCR tubes in a volume of 47 µl. Samples (3 µl) will be added to the reaction mix and mixed thoroughly.
10. Reaction plates will be loaded into a PE Applied Biosystems 7700 Sequence Detector, and a run cycle will be initiated using the manufacturer's recommended PCR conditions.
11. A standard curve prepared from known copy numbers of DNA isolated from BCBL-1 cells and amplified will be run with each plate in order to quantitate the DNA copy number in each original sample. The efficacy of the compound will be determined by comparing DNA copy numbers from test wells with those of control wells.
12. Twenty microliters of Cell Titer Aqueous One Solution (Promega) will be added to each well of the toxicity plates. Plates will be incubated at 37°C in a humidified CO₂ incubator for 4 hours or until sufficient color development has occurred. Plates will be read on a VMax microtiter plate reader at a wavelength of 490/650 nm. Toxicity of the test compound will be determined by comparing the optical density of test wells with that of control wells.

### Results from Human Herpesvirus 8 experiments:

**Chronically HHV8-infected BCBL-1 cells were treated with Compound No. 4**

| Compound | IC₅₀ (µM) | TC₅₀ (µM) | TI | Comments |
|---|---|---|---|---|
| No.4 | 2.3 | 78.9 | 34 | Active |

The minimum inhibitory drug concentration which reduces the viral replication by 50% (IC₅₀) and the minimum drug concentration which inhibits cell growth by 50% (TC₅₀) are calculated by using a regression analysis program for semilog curve fitting. A therapeutic (selectivity) index (TI) for compound No. 4 is determined by dividing the TC₅₀ by the IC₅₀.

### Varicella-zoster virus experiments:

MRC-5 (human embryonic lung fibroblast) cells (ATCC Cat. No. CCL-171) are seeded in 24 well plates using Eagle's Minimum Essential Medium (EMEM) supplemented with 10% heat-inactivated fetal bovine serum (FBS), L-glutamine, sodium pyruvate, non-essential amino acids, penicillin, and streptomycin. The plates are then incubated overnight at 37°C and 5% CO₂. The following day, media is aspirated and approximately 50-100 pfu (plaque forming units) of VZV strain ELLEN is added to the wells of each plate in a volume of 200 µl of assay media (EMEM supplemented with 5% heat-inactivated FBS, L-glutamine, sodium pyruvate, non-essential amino acids, penicillin, and streptomycin). The remaining wells of each plate serve as cellular control wells and receive 200 µl of assay media without virus. The virus is allowed to adsorb onto the cells for 1 hr at 37°C and 5% CO₂. Two dilutions of each test drug are prepared by diluting them in assay media containing 0.5% Methylcellulose. After the incubation period, 1 ml of each drug dilution is added to triplicate wells of a plate. Assay media (without drug) containing 0.5% Methylcellulose is added to the three cell control wells and to three virus control wells on each plate. The plates are incubated for 9-12 days to allow for plaque formation. The media is then aspirated from the wells and the cells are fixed and stained using 20% Methanol containing Crystal Violet. Plaques are enumerated by microscopic inspection and data is plotted as percent of virus contol.

Drug toxicity is determined in parallel by seeding MRC-5 cells in 96 well plates and adding drugs to triplicate wells at two dilutions. After a six day incubation period, cell viability is determined using CellTiter 96 Aqueous One Solution (Promega).

### Results from Varicella-zoster virus experiments:

**MRC-5 cells newly infected with VZV were treated with Compound No. 4**

| Compound | IC₅₀ (µM) | TC₅₀(µM) | TI | Comments |
|---|---|---|---|---|
| No.4 | 4.69 | 13.61 | 2.9 | Significant activity |

The minimum inhibitory drug concentration which reduces the CPE by 50% (IC₅₀) and the minimum drug concentration which inhibits cell growth by 50% (TC₅₀) are calculated by using a regression analysis program for semilog curve fitting. A therapeutic (selectivity) index (TI) for compound No. 4 is determined by dividing the TC₅₀ by the IC₅₀.

### Respiratory syncytial virus experiments:

A cytoprotection assay procedure using MTS is employed to evaluate compounds for antiviral activity against Respiratory Syncytial Virus (Long) in Vero (African green monkey, ATCC Cat. No. CCL-81) cells. The cells are pre-grown overnight at 37°C (2x10⁴/well in 96 well tissue culture plates) with 10% fetal Bovine serum containing Minimum Essential Medium. The positive control for the assay is Interferon-Gamma/Ribavirin. The infection medium contains reduced serum levels (2% FBS).

Using half log dilutions with high test starting at 100 µg/ml the drug dilutions are made and added to triplicate cultures in 0.1 ml volume and pre-titrated virus suspension is also added in 0.1 ml volume. Cell control containing medium alone, virus controls containing medium and virus, the drug toxicity controls containing medium and each drug concentrations are run simultaneously with the test samples. The plates are incubated at 37C° with 5% CO₂ until maximum CPE (cytopathogenic effect) observed in untreated virus controls. CPE inhibition is determined by dye uptake procedure (Celltiter- MTS). Celltiter reagent contains a novel tetrazolium (MTS) compound and an electron coupling reagent phenazine ethosulfate (PES). MTS-tetrazolium is bioreduced by cells into a colored formazan product that is soluble in tissue culture medium. This conversion is accomplished by NADPH or NADH produced by dehydrogenase enzymes in metabolically active cells. The amount of soluble formazan produced by cellular reduction of the MTS is measured by the absorbance at 490 nm. The quantity of formazan product as measured by the amount of 490 nm absorbance is directly proportional to the number of living cells in the culture thus allowing the rapid quantitative analysis of the inhibition of virus-induced cell killing by the test substances.

### Results from Respiratory syncytial virus experiments:

Vero cells newly infected with RSV were treated with Compound No. 4

| Compound | IC50 (µM) | TC50 (µM) | TI | Comment | IC95 (µM) | TC95 (µM) | TI | Comment |
|---|---|---|---|---|---|---|---|---|
| No.4 | 1,460 | 8,68 | 5,95 | Significant Activity | 2,96 | 30,20 | 10,20 | Active |

The minimum inhibitory drug concentration which reduces the viral replication by 50% (IC₅₀) and the minimum drug concentration which inhibits cell growth by 50% (TC₅₀) are calculated by using a regression analysis program for semilog curve fining. A therapeutic (selectivity) index (TI) for compound No. 4 is determined by dividing the TC₅₀ by the IC₅₀.

The inhibitory drug concentration which reduces the viral replication by 95% (IC₉₅) and the minimum drug concentration which inhibits cell growth by 95% (TC₉₅) are calculated by using a regression analysis program for semilog curve fitting. A therapeutic (selectivity) index (TI) for compound No. 4 is determined by dividing the TC₉₅ by the IC₉₅.

## Claims

1. Use of the compound of formula 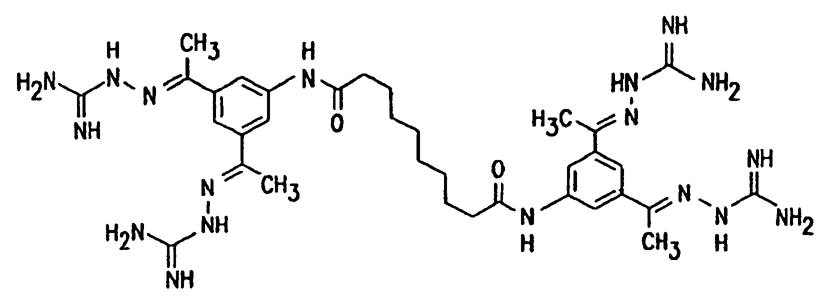 (N,N'-bis (3,5-diacetylphenyl) decane diamide tetrakis (amidinohydrazone)),
or a salt thereof for the manufacture of a medicament for the treatment of infections caused by drug-resistant HIV-1 strains or by Lamivudine-resistant Hepatitis B virus.

2. Use according to claim 1 where the HIV-1 strain is resistant to all currently available anti-HIV drugs.

3. Use according to claim 1 where the HIV-1 strain is resistant to Protease Inhibitors.

4. Use according to claim 1 where the HIV-1 strain is resistant to Non-Nucleoside Reverse Transcriptase Inhibitors.

5. Use according to claim 1 where the HIV-1 strain is resistant to Nucleoside Reverse Transcriptase Inhibitors and Protease Inhibitors.

6. Use according to claim 1 where the HIV-1 strain is a macrophage-tropic strain.

7. Use according to claim 1 where the HIV-1 strain causes Acquired Immunodeficiency Syndrome.

8. Use according to claim 1 where the HBV strain causes chronic liver inflammation, liver cirrhosis, or liver cancer.

9. Use according to claim 1 wherein the compound of the disclosed formula or pharmaceutically acceptable salts thereof is to be administered to a patient in need preferably in a dosage corresponding to an effective concentration in the range of 0.01-10 µM.

10. Use according to claim 1 wherein the compound of the disclosed formula or pharmaceutically acceptable salts thereof is to be administered to a patient in need most preferably in a dosage corresponding to an effective concentration in the range of 0.1-5 µM.

## Patentansprüche

1. Verwendung der Verbindung der Formel 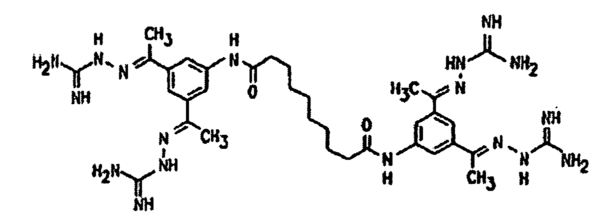 N,N'-Bis(3,5-diacetylphenyl)decandiamid-Tetrakis (Amidinohydrazon)
oder eines Salzes davon zur Herstellung eines Medikaments zur Behandlung von Infektionen, die von arzneimittelresistenten HIV-1-Stämmen oder von dem Hepatitis-B-Virus mit Lamivudin-Resistenz verursacht werden.

2. Verwendung nach Anspruch 1, wobei der HIV-1-Stamm gegenüber allen derzeit erhältlichen Anti-HIV-Arzneimitteln resistent ist.

3. Verwendung nach Anspruch 1, wobei der HIV-1-Stamm gegenüber Protease-Hemmern resistent ist.

4. Verwendung nach Anspruch 1, wobei der HIV-1-Stamm gegenüber nicht nukleosidalen Reverse-Transkriptase-Hemmern resistent ist.

5. Verwendung nach Anspruch 1, wobei der HIV-1-Stamm gegenüber nukleosidalen Reverse-Transkriptase-Hemmern resistent ist.

6. Verwendung nach Anspruch 1, wobei der HIV-1-Stamm ein auf Makrophagen gerichteter Stamm ist.

7. Verwendung nach Anspruch 1, wobei der HIV-1-Stamm das erworbene Immunschwächesyndrom verursacht.

8. Verwendung nach Anspruch 1, wobei der HBV-Stamm chronische Leberentzündung, Leberzirrhose oder Leberkrebs verursacht.

9. Verwendung nach Anspruch 1, wobei die Verbindung der offenbarten Formel oder pharmazeutisch unbedenkliche Salze davon vorzugsweise in einer Dosis an einen bedürftigen Patienten verabreicht wird, die einer wirksamen Konzentration im Bereich von 0,01-10 µM entspricht.

10. Verwendung nach Anspruch 1, wobei die Verbindung der offenbarten Formel oder pharmazeutisch unbedenkliche Salze davon vorzugsweise in einer Dosis an einen bedürftigen Patienten verabreicht wird, die einer wirksamen Konzentration im Bereich von 0,1-5 µM entspricht.

## Revendications

1. Utilisation du composé de formule : 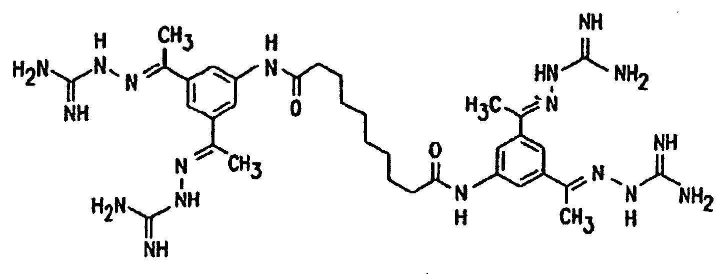 (N,N'-bis (3,5-diacétylphényl) décane diamine tétrakis (aminohydrazone)),
ou d'un sel de celui-ci pour la fabrication d'un médicament destiné au traitement d'infections provoquées par des souches du VIH-1 résistantes aux médicaments ou par le virus de l'hépatite B résistant à la lamivudine.

2. Utilisation selon la revendication 1, dans laquelle la souche de VIH-1 est résistante à tous les médicaments anti-VIH actuellement disponibles.

3. Utilisation selon la revendication 1, dans laquelle la souche de VIH-1 est résistante aux inhibiteurs de la protéase.

4. Utilisation selon la revendication 1, dans laquelle la souche de VIH-1 est résistante aux inhibiteurs de la transcriptase inverse non nucléosidiques.

5. Utilisation selon la revendication 1, dans laquelle la souche de VIH-1 est résistante aux inhibiteurs de la transcriptase inverse nucléosidiques et aux inhibiteurs de la protéase.

6. Utilisation selon la revendication 1, dans laquelle la souche de VIH-1 est une souche à tropisme pour les macrophages.

7. Utilisation selon la revendication 1, dans laquelle la souche de VIH-1 est responsable du syndrome de l'immunodéficience acquise.

8. Utilisation selon la revendication 1, dans laquelle la souche de HBV entraîne une inflammation chronique du foie, une cirrhose hépatique ou un cancer du foie.

9. Utilisation selon la revendication 1, dans laquelle le composé de la formule décrite ou des sels pharmaceutiquement acceptables de celui-ci seront administrés à un patient les nécessitant, de préférence en une dose correspondant à une concentration efficace dans la plage de 0,01 à 10 µM.

10. Utilisation selon la revendication 1, dans laquelle le composé de la formule décrite ou des sels pharmaceutiquement acceptables de celui-ci seront administrés à un patient les nécessitant, de manière davantage préférée en une dose correspondant à une concentration efficace dans la plage de 0,1 à 5 µM.
